# EUROPEAN PATENT APPLICATION

(11) **EP 1 939 176 A1**
(43) Date of publication of application: **02.07.2008**
(21) Application number: 06126976.7
(22) Date of filing: 22.12.2006
(51) Int. Cl.: C07D 209/14, A61K 31/404

(54) **Salts of Tegaserod**

(71) Applicant: Novartis AG, 4056 Basel (CH)
(72) Inventor: The designation of the inventor has not yet been filed
(74) Representative: McLean, Craig Sutherland

(57) **Abstract**

The present invention relates to novel salt forms forms of tegaserod, their crystalline forms and their use in pharmaceutical compositions useful in the treatment of irritable bowel syndrome, gastro-esophageal reflux disease, functional dyspepsia, chronic constipation or diarrhea, and their production.

## Description

This invention relates to novel salts of the partial 5-HT₄ receptor 3-(5-methoxy-1 H-indol-3-yl-methylene)-N-pentylcarbazimidamide (I), which has the INN name tegaserod.

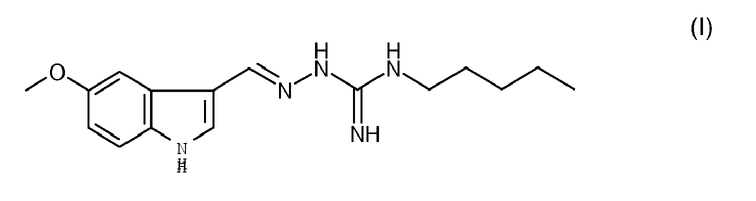

In addition, the invention relates to pharmaceutical compositions comprising the salts of the invention, to uses of said salts in medicine and to processes to obtain them. Such pharmaceutical compositions are particularly relevant to the treatment of conditions for which a partial agonist of 5-HT₄ receptors is required, such as gastrointestinal disorders (Gl disorders) which affect the gastrointestinal tract from the mouth to the anus. Such GI disorders may be but are not limited to, heartburn, bloating, postoperative ileus, abdominal pain and discomfort, early satiety, epigastric pain, nausea, vomiting, burbulence, regurgitation, intestinal pseudoobstruction, anal incontinence, irritable bowel syndrome (IBS), gastro-esophageal reflux disease (GERD), functional dyspepsia, chronic constipation or diarrhea, gastroparesis, e.g. diabetic gastroparesis, ulcerative colitis, Crohn's disease, ulcers, gastrointestinal hyperactivity, and relaxing effect on intestinal smooth muscle cells and the visceral pain associated therewith.

European patent 0505322 describes a family of aminoguanidines derivatives including tegaserod and pharmaceutically acceptable salts thereof as 5-HT₄ receptor agonists. The pharmaceutically acceptable salts include acid addition salts, specifically the hydrochloride, hydrobromide, hydrofluoride, sulphate or bisulphate, phosphate or hydrogen phosphate, acetate, besylate, citrate, fumarate, gluconate, lactate, maleate, malonate, malate, mesylate, succinate and tartrate salts. The hydrogen maleate salt form has been marketed world-wide for medical use under the Trade marks ZELMAC and ZELNORM.

It is an object of the present invention to provide salts of tegaserod which exhibit improved properties as medicaments, for example stability, solubility, crystallinity, formulation ability, bioavailability or the like.

Thus as a first aspect, the invention provides a salt form of tegaserod prepared from an acid selected from: acetic acid; L-malic acid; naphtalene 1,5 disulfonic acid; adipic acid phosphoric acid nicotinic acid L-ascorbic acid; succinic acid; oxalic acid; citric acid; orotic acid; aspartic acid; fumaric acid; thiocyanic acid; propionic acid; galactaric acid; salycilic acid; L-pyroglutamic acid; glutaric acid; benzenesulfonic acid; p-toluenesulfonic acid; glycolic acid; benzoic acid; camphor 10 sulfonic acid; glucuronic acid; (+) camphoric acid; formic acid; hippuric acid; ethansulfonic acid; 4-acetamidobenzoic; α-oxo-glutaric; gentisic acid; lactobionic acid; DL-lactic acid; malonic acid; sulfuric acid; squaric acid; deoxycholic acid; 1-hydroxy-naphtoic acid; phtalic acid; vanillic acid; methansulfonic acid; dichloroacetic acid; isobutyric acid; nitric acid; carbonic acid; and emboic acid.

### Suitable salt forms of tegaserod are selected from:

Tegaserod glycolate; Tegaserod D,L-lactate; Tegaserod L-lactate; Tegaserod acetate; Tegaserod citrate; Tegaserod sulfate; Tegaserod hydrogenmalate; Tegaserod malate; Tegaserod D-gluconate; Tegaserod glucuronate; Tegaserod salicylate; Tegaserod gentisate; Tegaserod hippurate; Tegaserod succinate; Tegaserod fumarate; Tegaserod adipate; Tegaserod thiocyanate; Tegaserod aspartate; Tegaserod malonate; Tegaserod galactarate; Tegaserod oxoglutarate; Tegaserod pidolate; Tegaserod glutarate; Tegaserod benzoate; Tegaserod oxalate; Tegaserod esylate; Tegaserod Mesylate; Tegaserod formate; Tegaserod quadrate; Tegaserod propionate; Tegaserod dichloroacetate; Tegaserod acetamidobenzoate; Tegaserod besylate; Tegaserod pyruvate; Tegaserod tosylate; Tegaserod isobutyrate; Tegaserod caprylate; Tegaserod napadisilate; Tegaserod xinafoate; Tegaserod pamoate; and Tegaserod nitrate.

More suitable salt forms of tegaserod are selected from: Tegaserod pidolate; Tegaserod formate; and Tegaserod oxoglutarate.

As a further aspect of the present invention, there is provided solvates of the salts of the present invention. Suitable solvates include monohydrates.

Suitable solvates of salt forms of tegaserod are selected from:
Tegaserod hydrogencitrate monohydrate; Tegaserod sulfate monohydrate; Tegaserod D-gluconate hemihydrate; and Tegaserod galactarate monohydrate.

As a further aspect of the present invention, there is provided salts of the invention in preferred solid forms, e.g. amorphous, semi-amorphous or crystalline forms. The crystalline forms of the salts of the present may be characterized by the major peaks of an x-ray powder diffraction spectrum or a raman spectroscopy spectrum, as shown in the examples hereinafter.

Solid forms, e.g. crystal modifications, and solvates of the salt forms of tegaserod differ with respect to their thermodynamic stability, in their physical parameters, such as the absorption pattern in an Infrared Absorption Spectrometry (IR), the phase transition signal in Differential Scanning Calorimetry (DSC), the powder diffraction pattern in X-ray and in their preparation processes.

Specific crystal forms of the invention may exhibit advantageous properties, such as being well-defined, being more stable when exposed to heat and more stable in the presence of water. Certain crystal forms can under certain conditions, e.g. heat or water containing organic solvents, completely or partly be converted into other crystal forms. Thus, the converted crystal form is normally characterized as being the thermodynamically more stable form. Similarly properties are observed for solvates, whereby solvate forms can by modified to other solvate forms by methods well known to those skilled in the art.

The invention relates to crystal forms of the salts of the invention. Preferably the crystal forms are of greater than 90%, more preferably 95%, more preferably 96%, more preferably 97%, more preferably 98%, more preferably 99% polymorphic purity as determined, e.g. by X-ray powder diffraction, Raman spectroscopy and IR spectrum. The invention relates preferably to essentially pure forms of the crystals of the invention. The term "essentially pure" as used herein means that less than about 5% and preferably less than about 2% by weight in the preparation, e.g. as a pure drug substance or in a pharmaceutical composition is not the desired crystal form (but another form e.g. another crystal form or any of the solvates), based upon 100% total weight of the salt form in the preparation, e.g. as pure drug substance or in a pharmaceutical composition.

Crystal forms of the salts of tegaserod of the present invention may be prepared by crystallization or recrystallization of any form, or mixtures of any forms of the salt in a solution comprising e.g. water and appropriate solvent.

A preferred embodiment of the invention relates to a process for the preparation of a crystal form of a tegaserod salt of the present invention, comprising the step of crystallizing any form, or mixtures of any forms of the salt in a solution comprising a suitable solvent, e.g. an organic solvent and optionally water.

Working up the crystallized form of a tegaserod salt according to the present invention and purification thereof thus obtained may be carried out in accordance to known procedures.

The invention also relates to pharmaceutical preparations comprising the salt forms of tegaserod of the present invention. The invention relates in particular to corresponding pharmaceutical preparations for the treatment of disorders in which 5-HT₄ agonsits and partial agonists are implicated, e.g. irritable bowel syndrome, gastro-esophageal reflux disease, functional dyspepsia, chronic constipation or diarrhea and sub-indications thereof. The invention relates to the use of the salt forms of the present invention for the preparation of pharmaceutical preparations, in particular for the treatment of irritable bowel syndrome, gastro-esophageal reflux disease, functional dyspepsia, chronic constipation or diarrhea and subindications thereof.

The salt forms of the present invention can be used, for example, in the form of pharmaceutical preparations which comprise a therapeutically effective amount of the active ingredient, if desired together with inorganic or organic, solid or liquid, pharmaceutically usable carriers, which are suitable for enteral, for example oral, or parenteral administration. Furthermore, the salt forms of the present invention can be used in the form of preparations which can be administered parenterally or of infusion solutions. The pharmaceutical preparations may be sterilized and/or may comprise excipients, for example preservatives, stabilizers, wetting agents and/or emulsifiers, solubilizers, salts for regulating the osmotic pressure and/or buffers. The present pharmaceutical preparations comprise from about 0.1 % to 100%, in particular from about 1% to about 50%, of lyophilisates to about 100% of the active ingredient.

The invention also relates to the use of a salt form of the present invention as a drug, preferably in the form of pharmaceutical preparations. The dosage may depend on various factors, such as method of administration, species, age and/or individual condition. The doses to be administered daily are between about 0.0016 and about 16 mg/kg in the case of oral administration, and preferably between about 1.2 mg and about 120 mg, more preferably about 12 mg, e.g. twice daily a 6 mg for warm-blooded species having a body weight of about 70 kg.

### Brief description of the Figures:

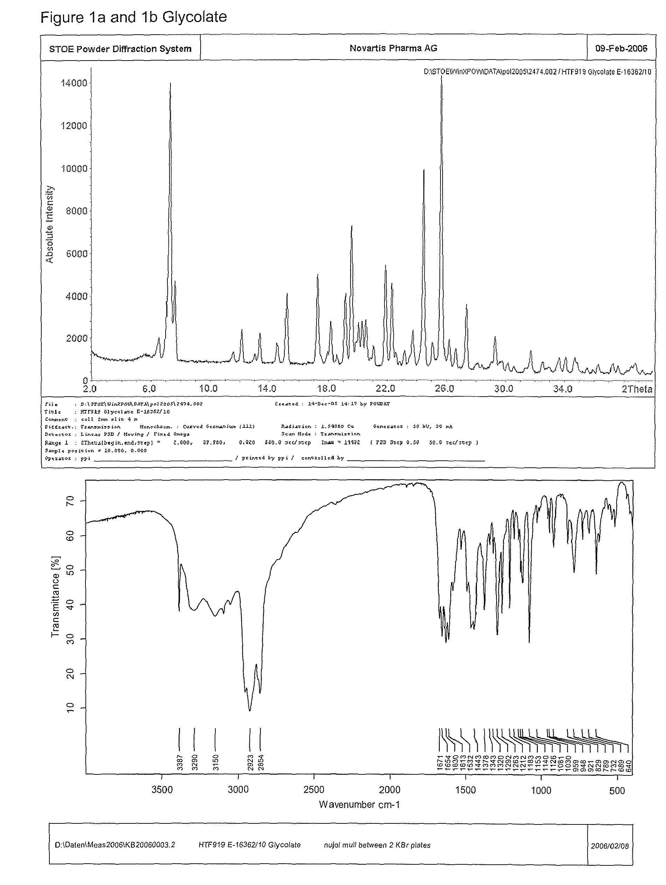

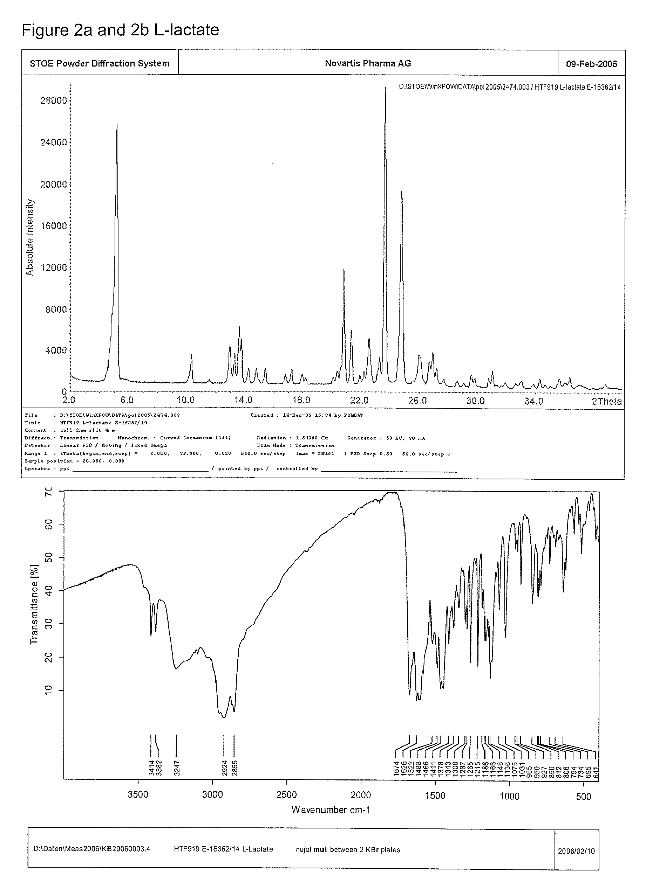

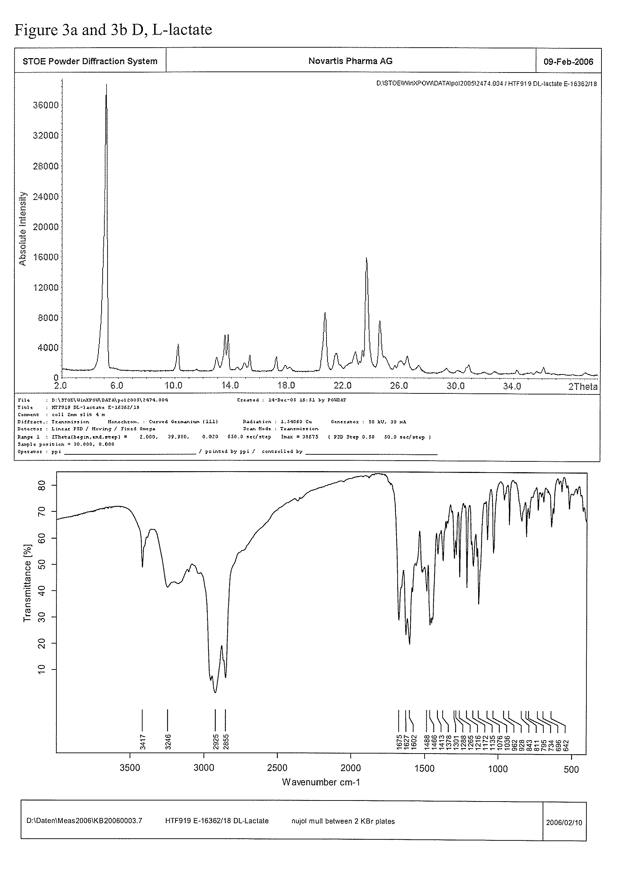

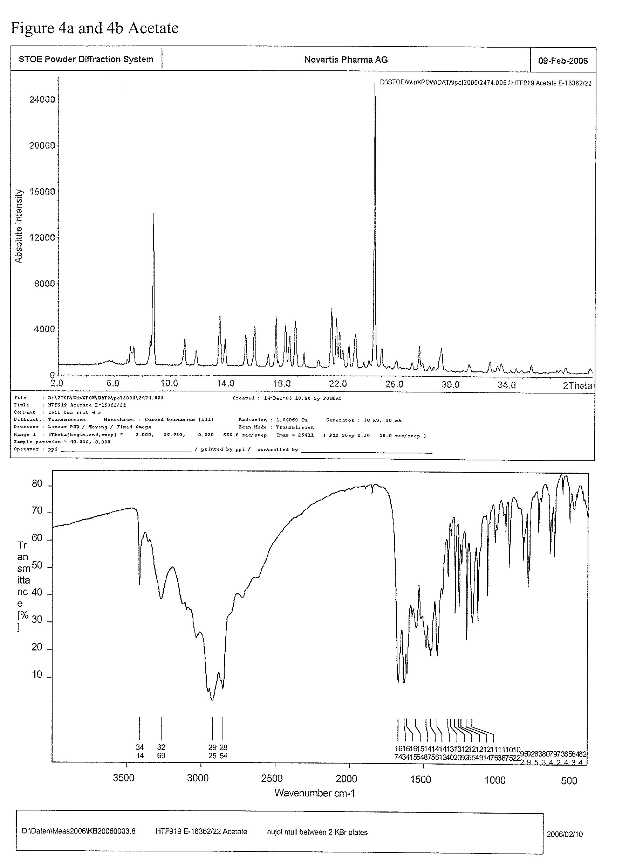

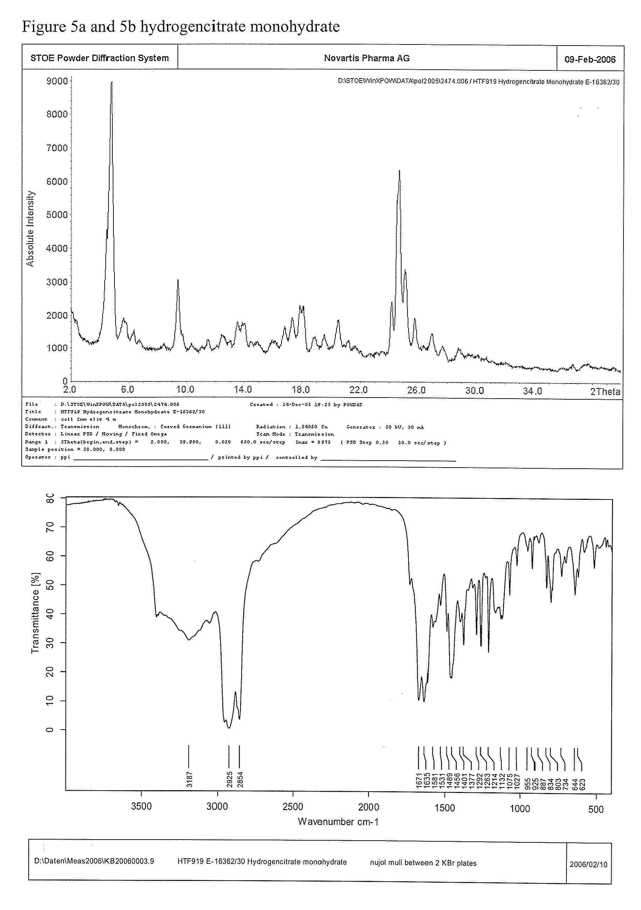

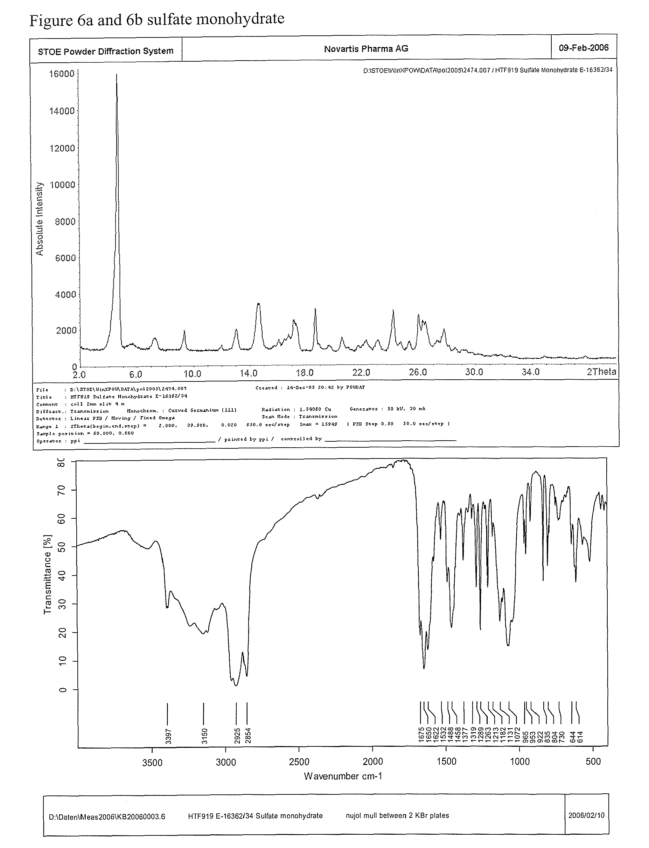

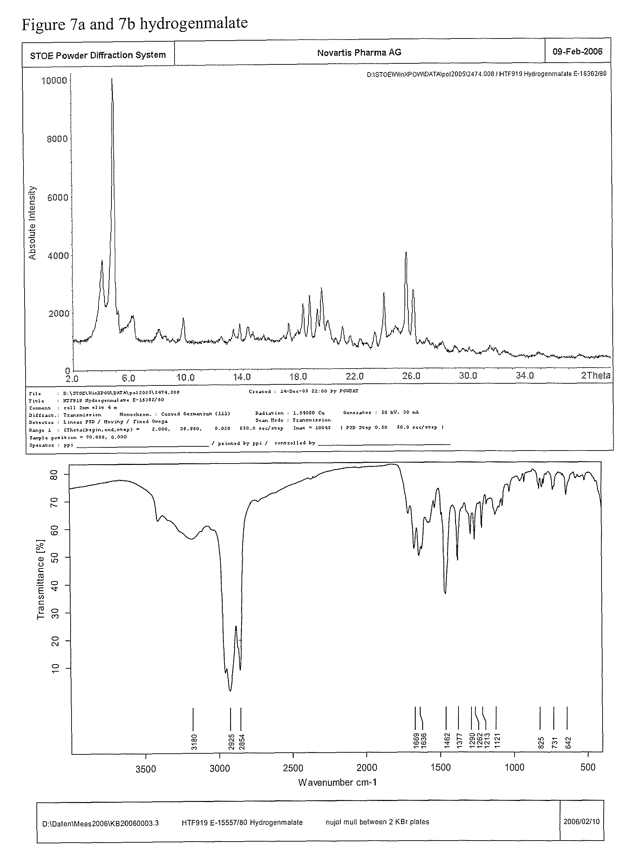

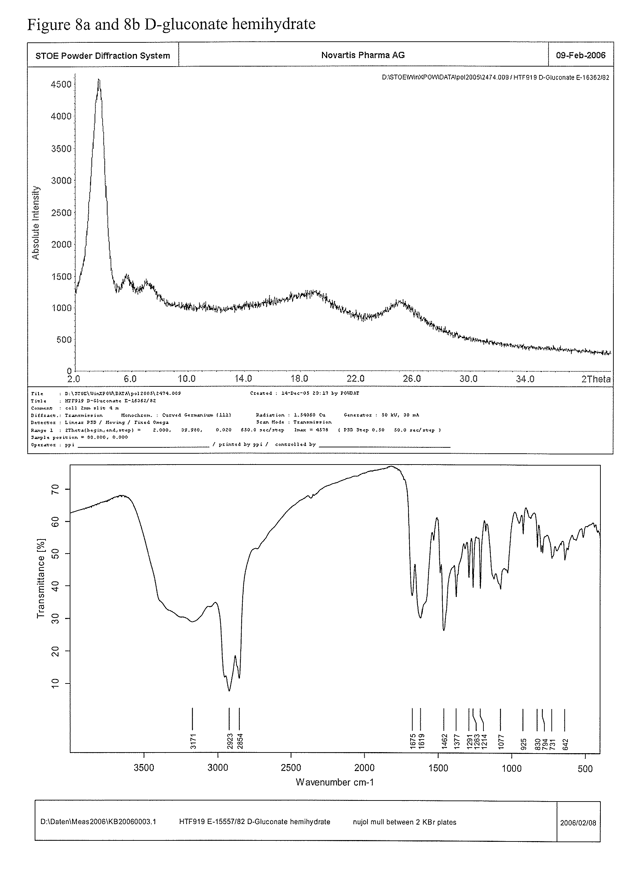

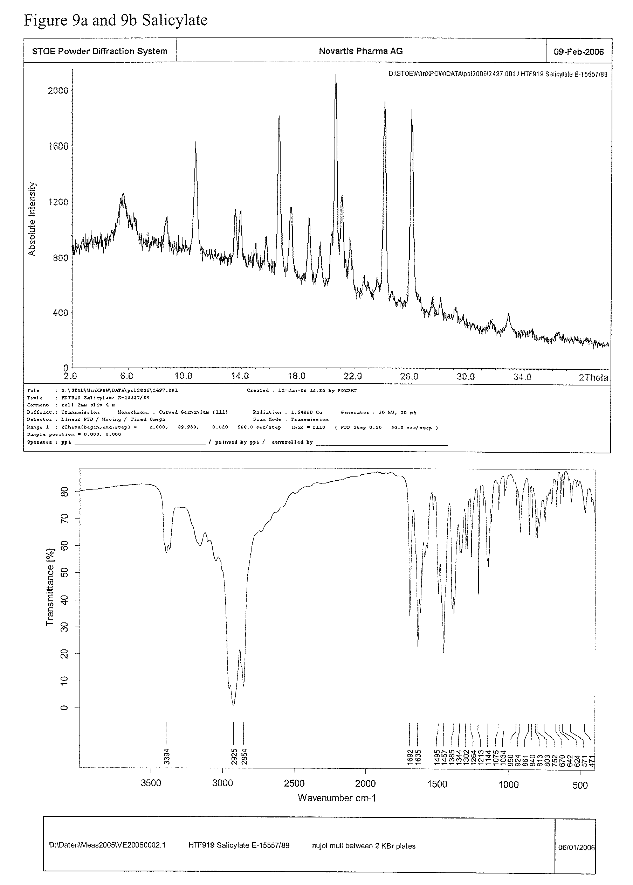

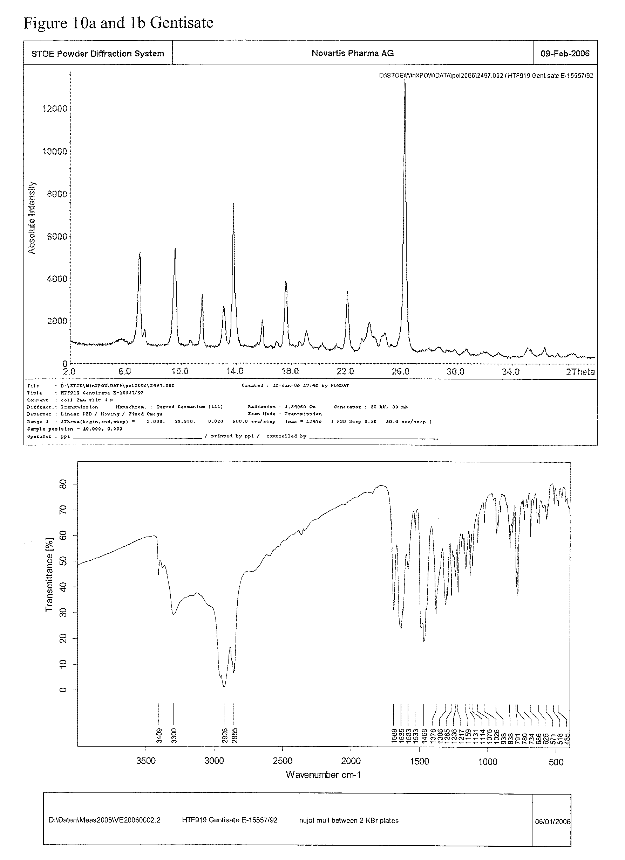

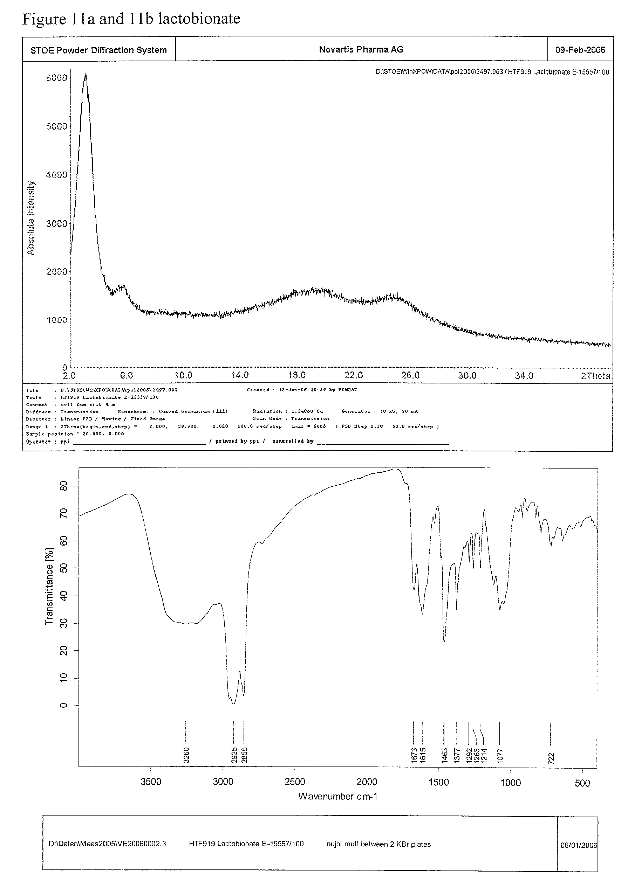

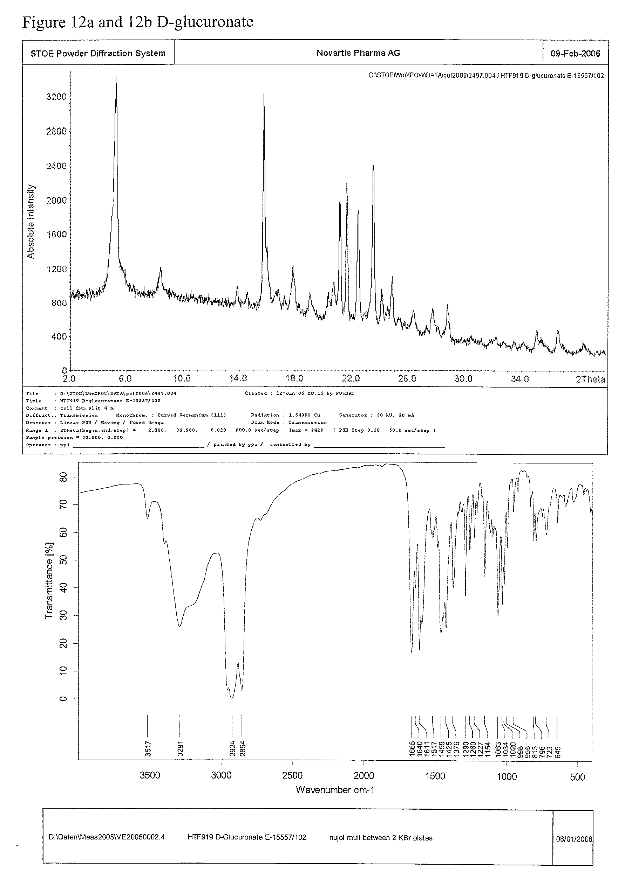

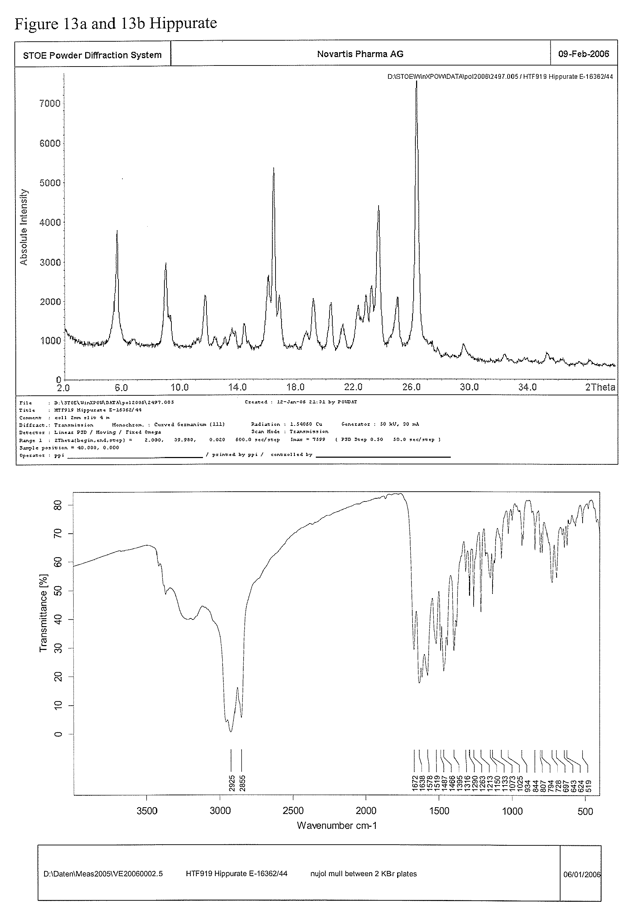

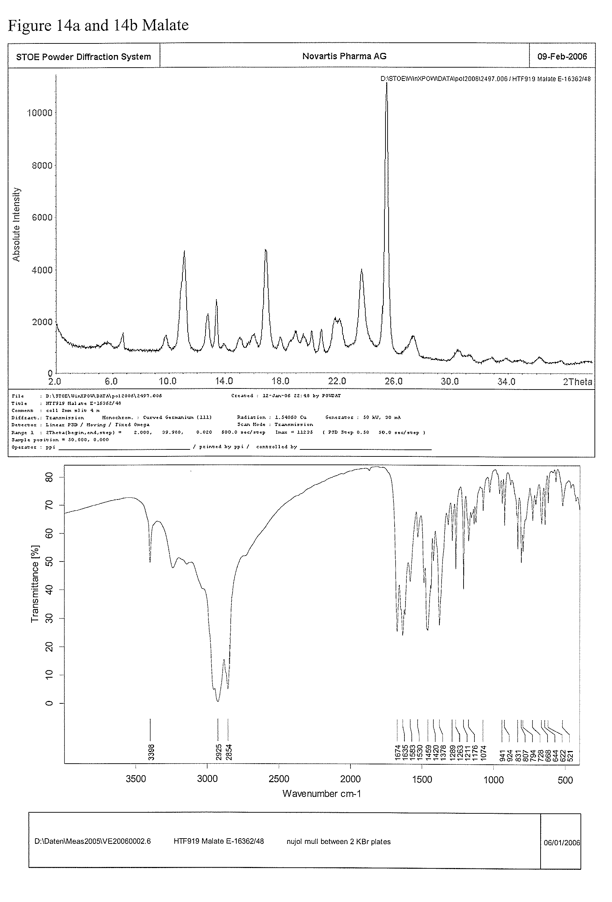

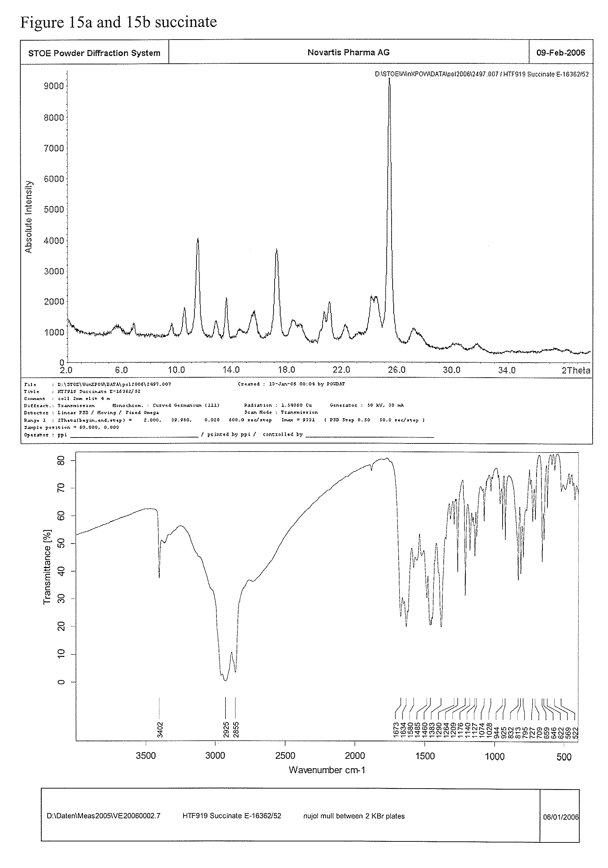

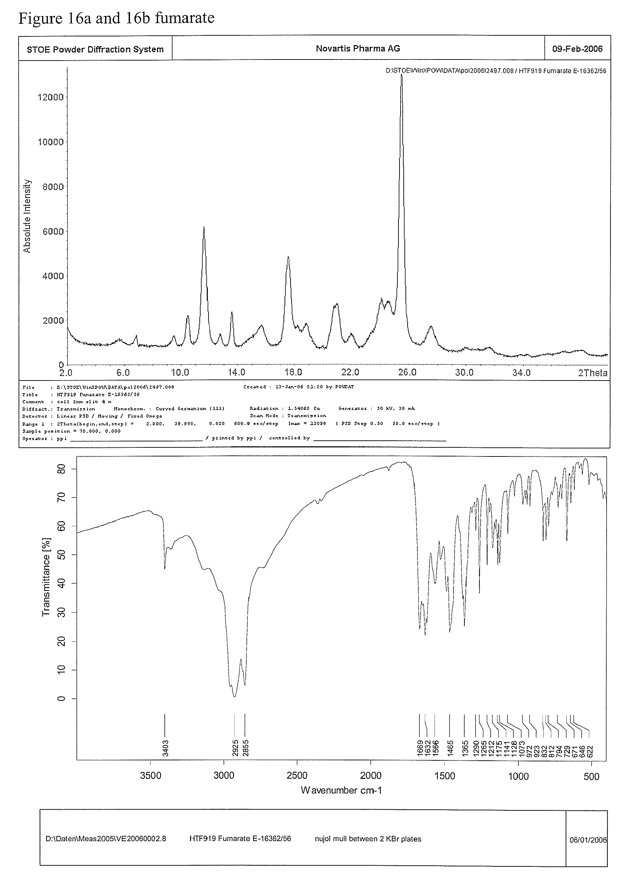

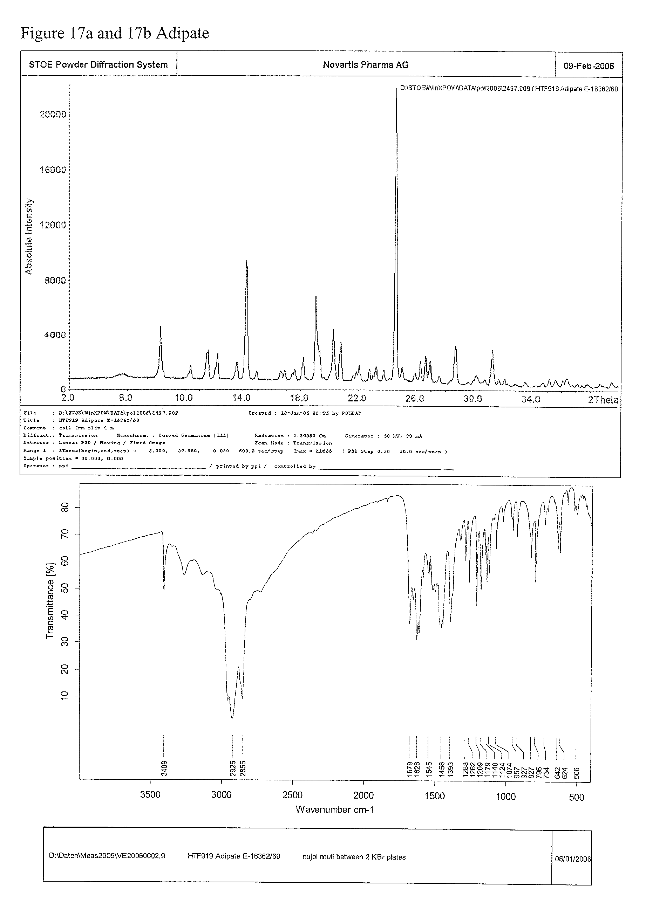

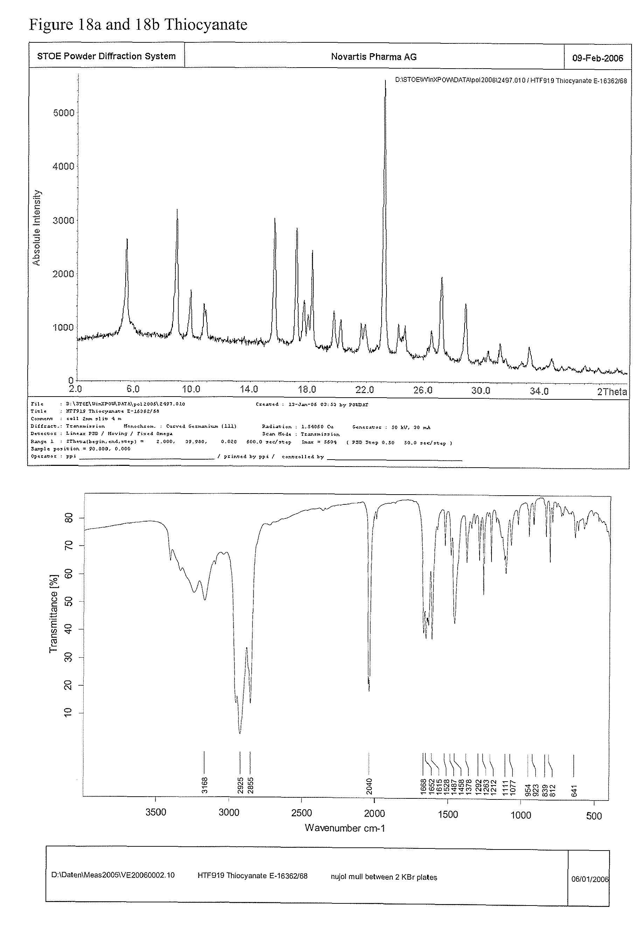

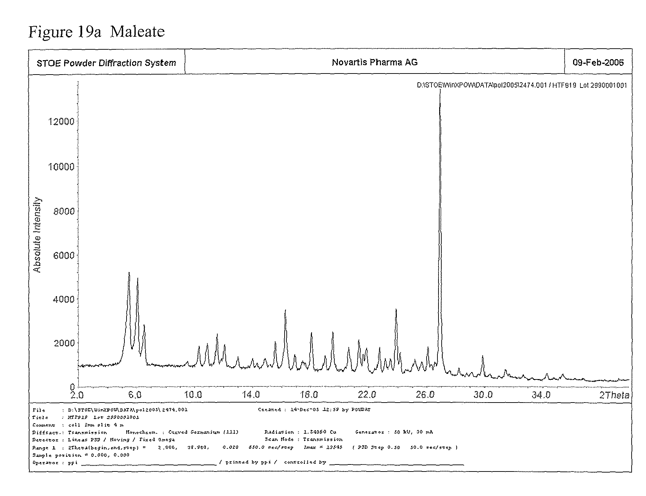

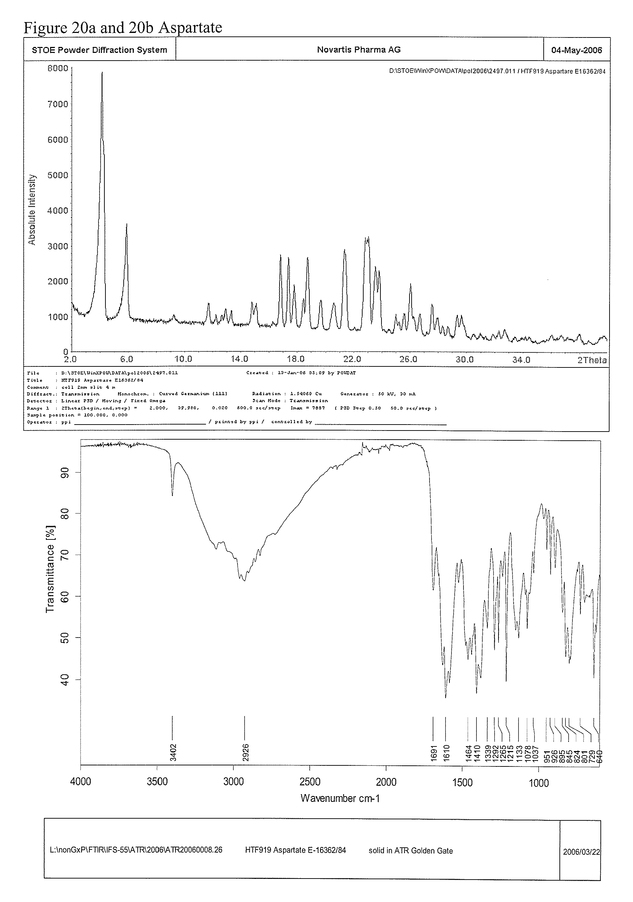

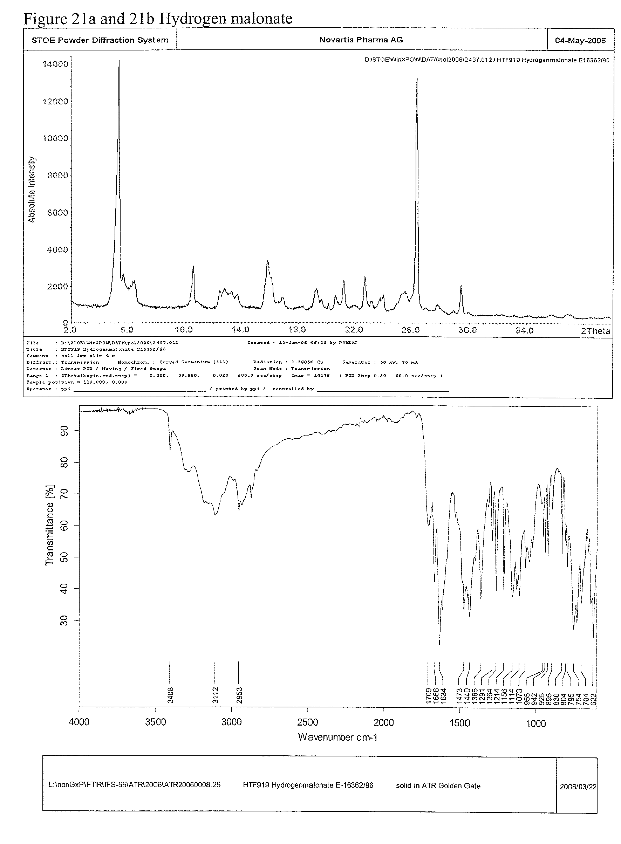

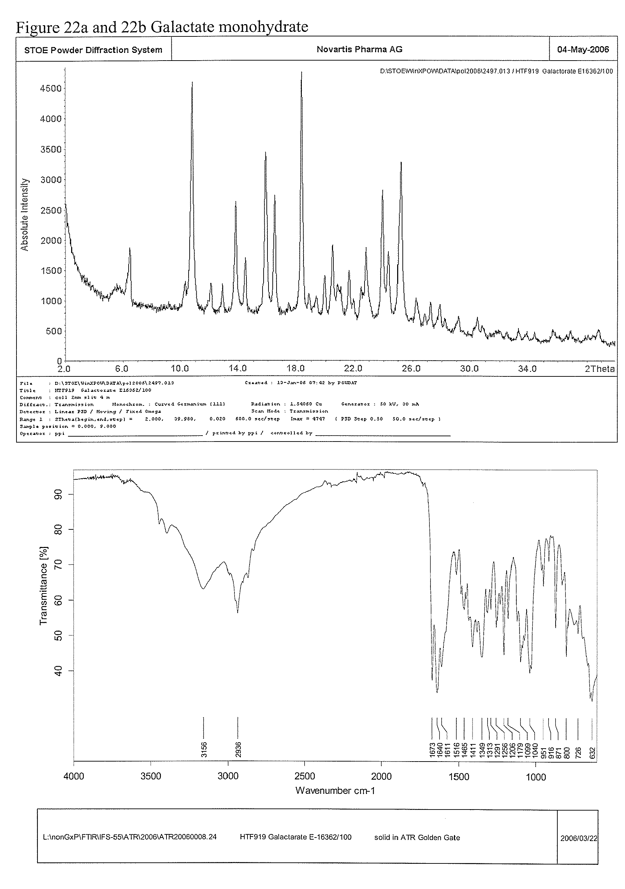

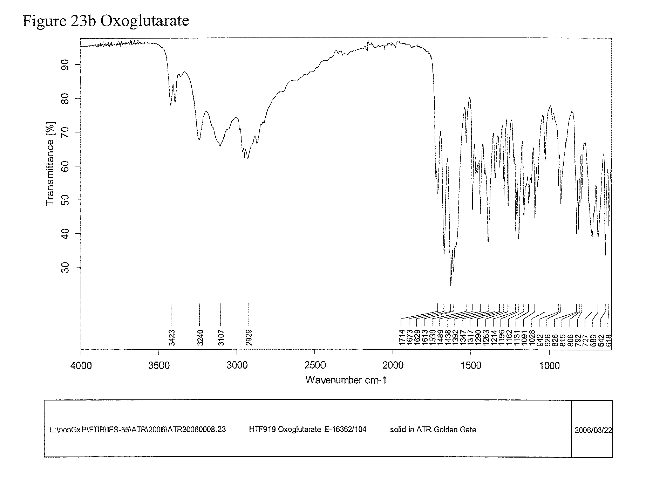

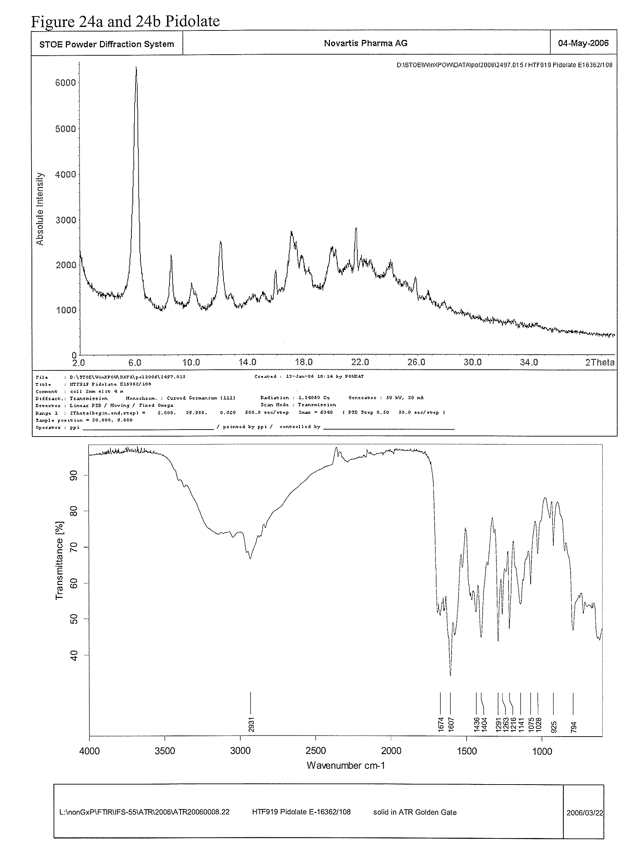

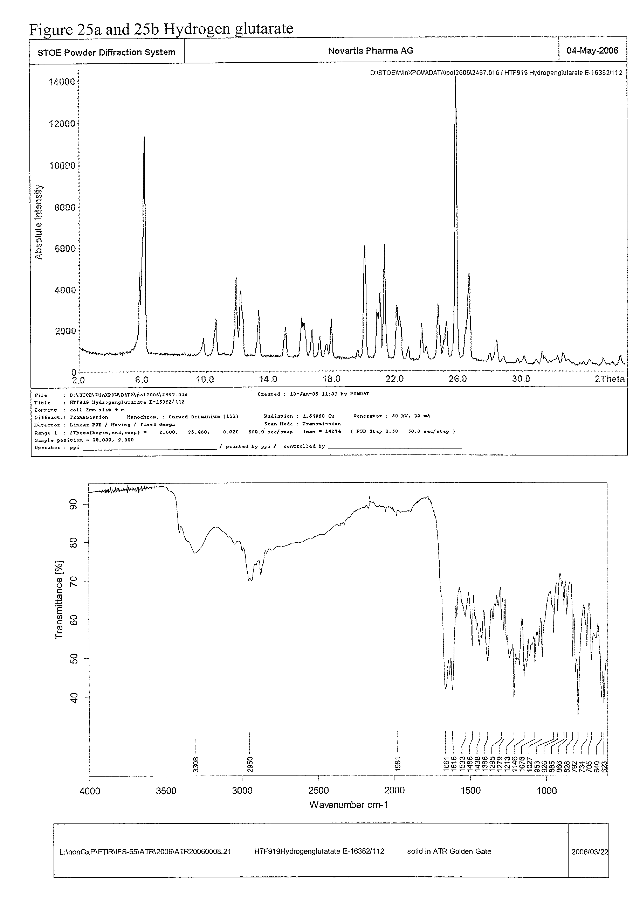

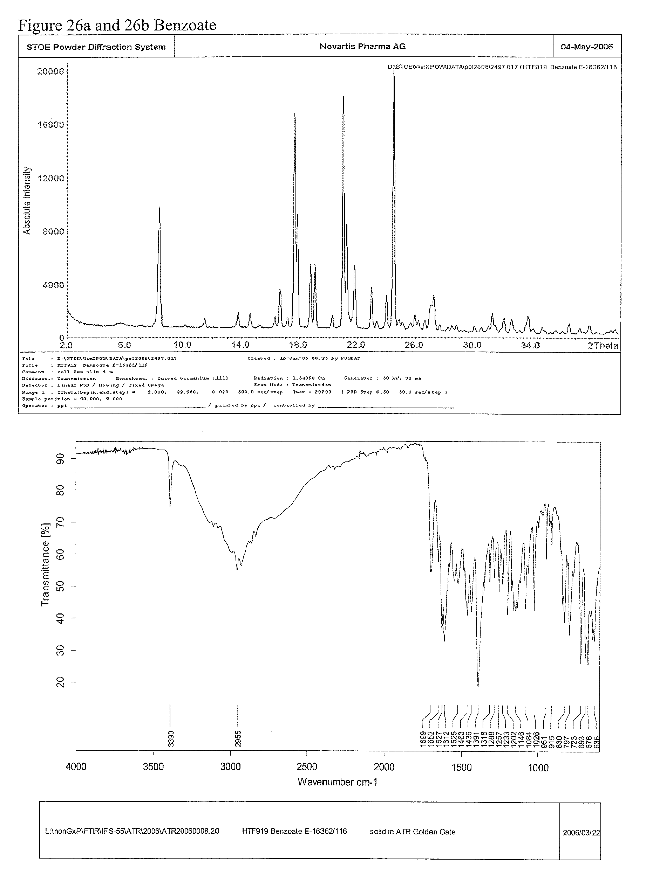

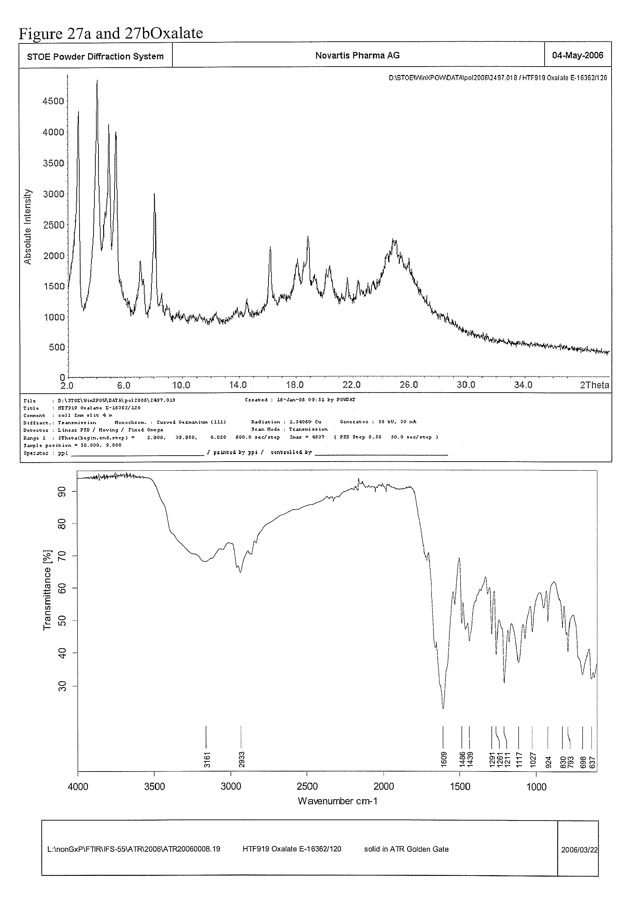

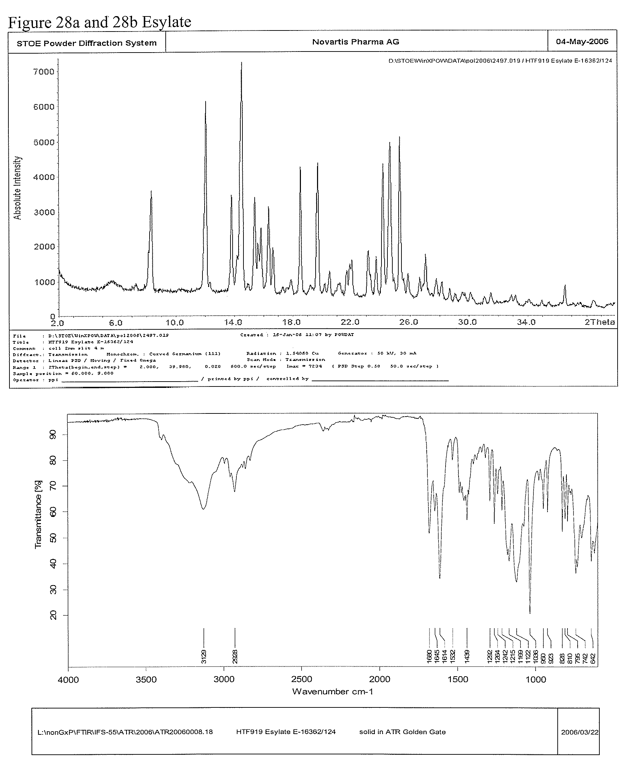

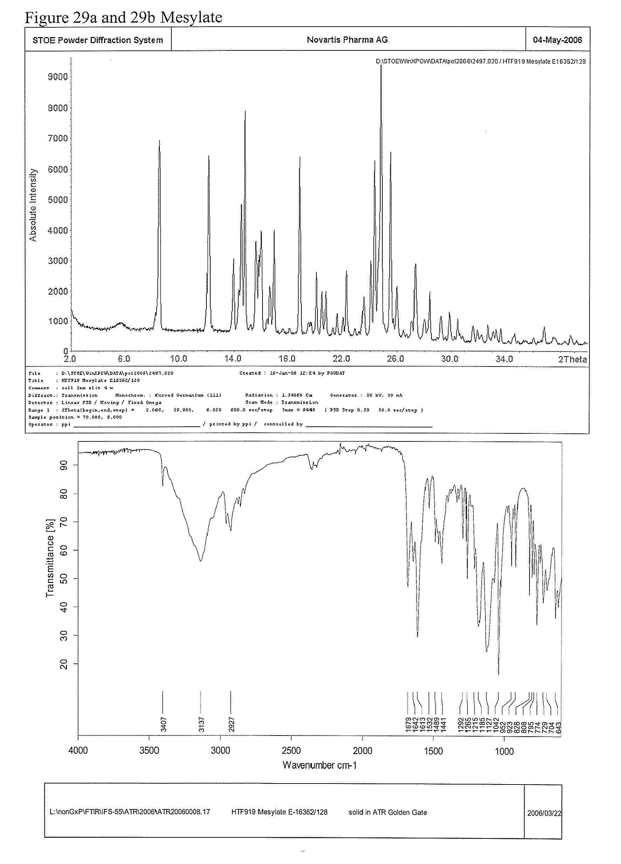

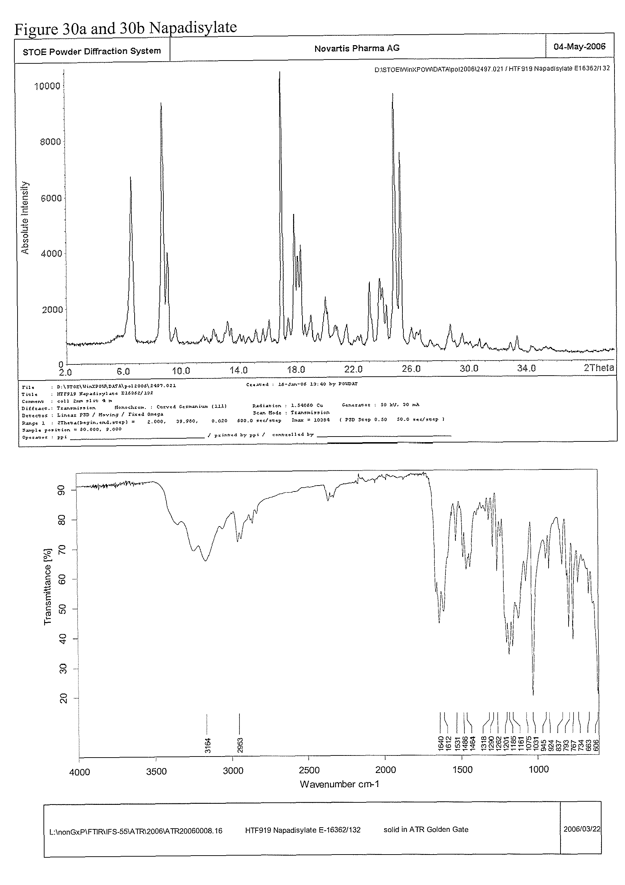

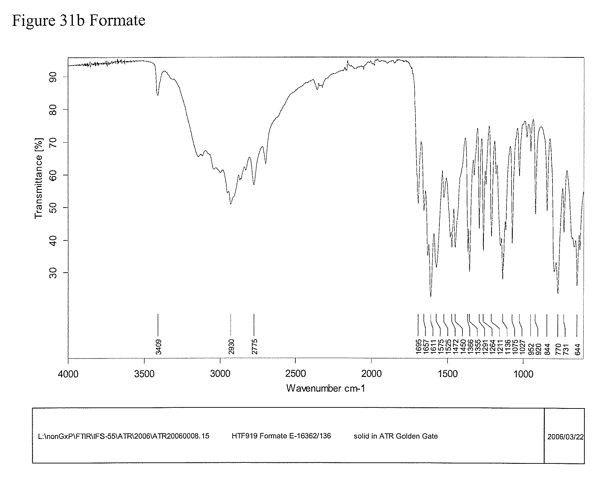

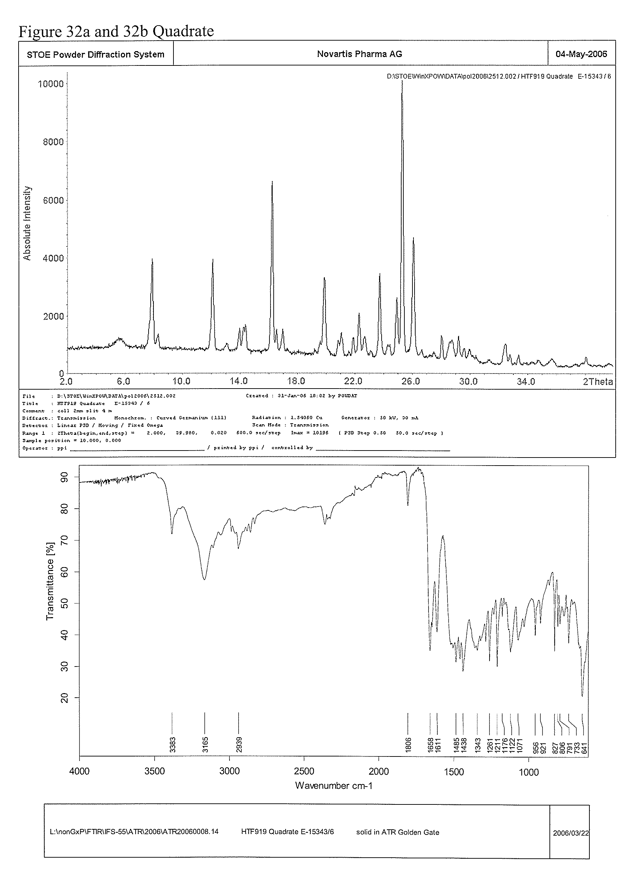

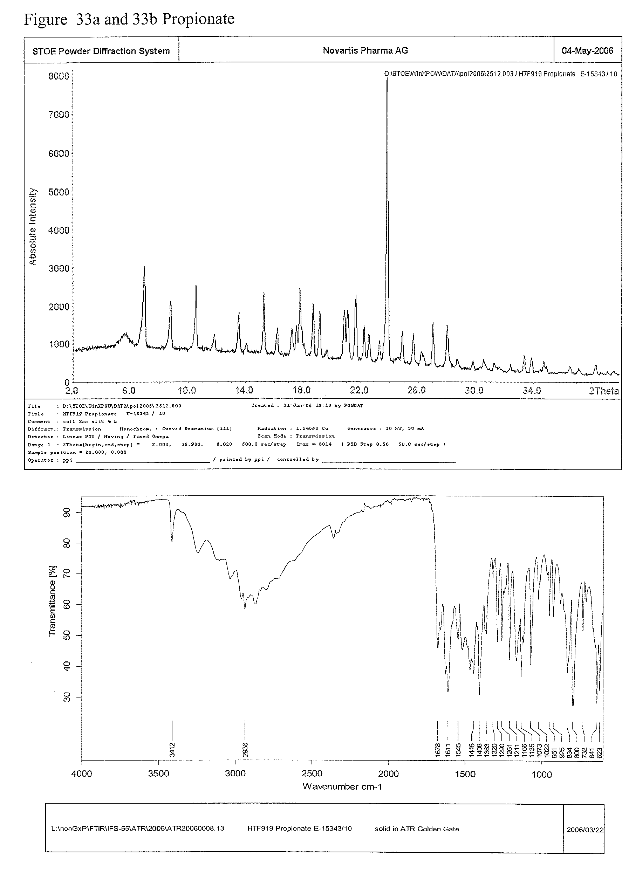

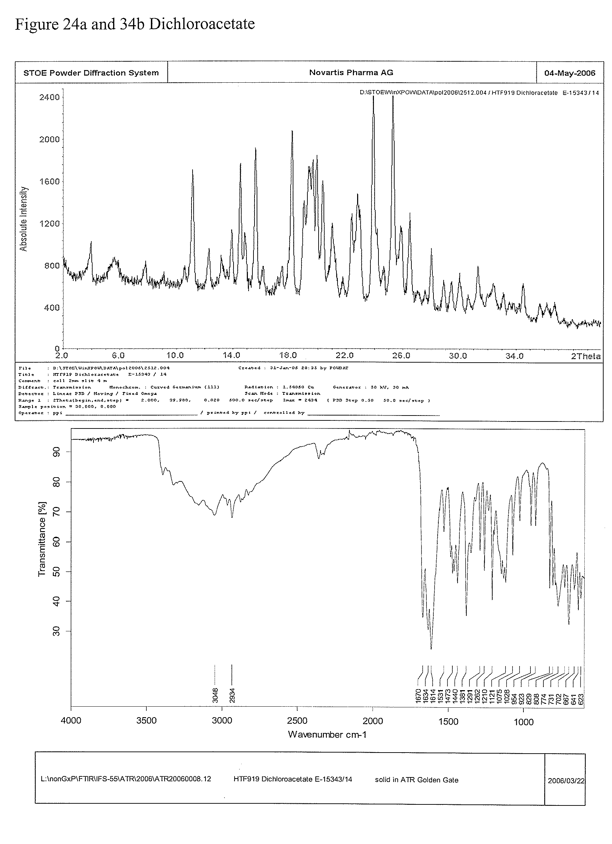

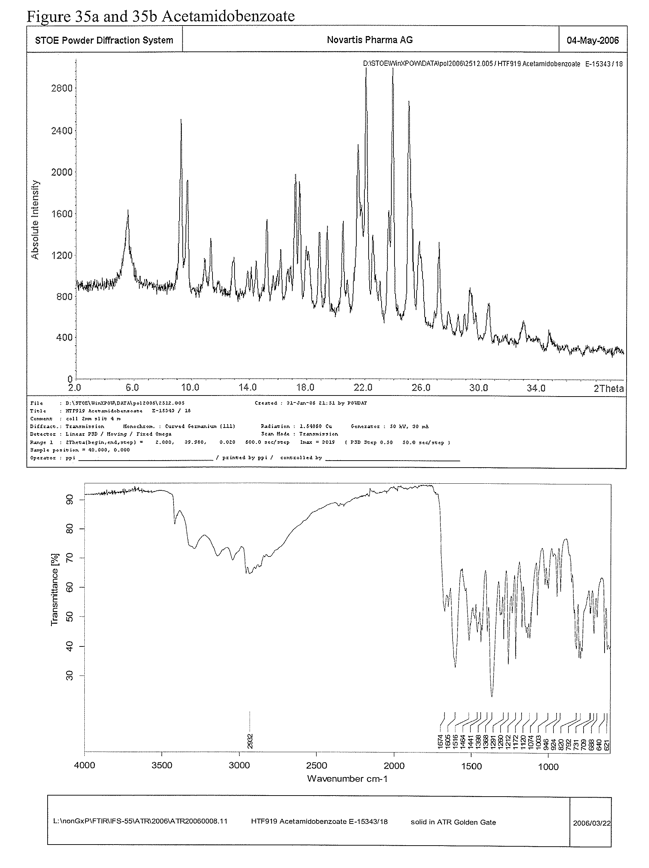

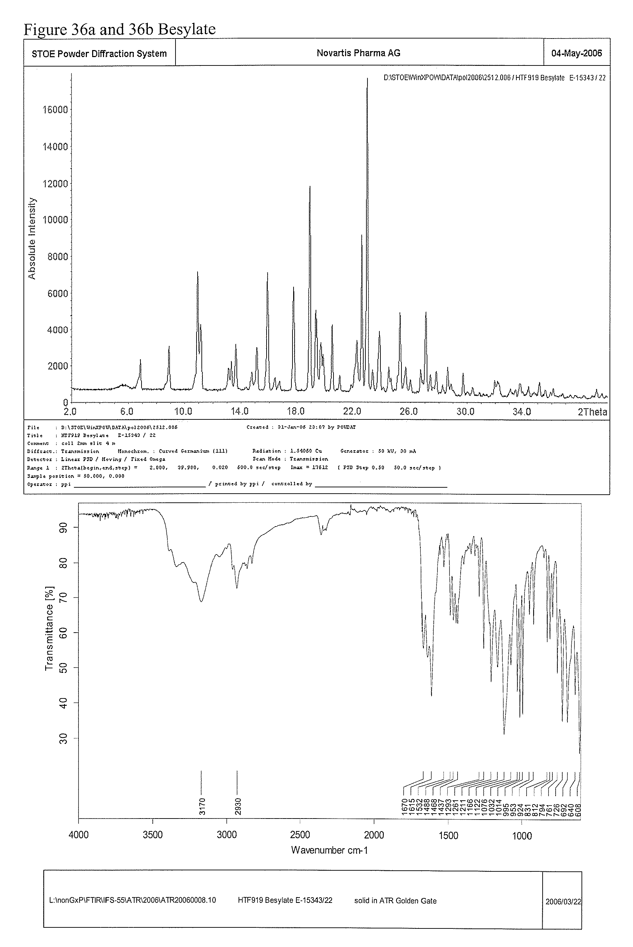

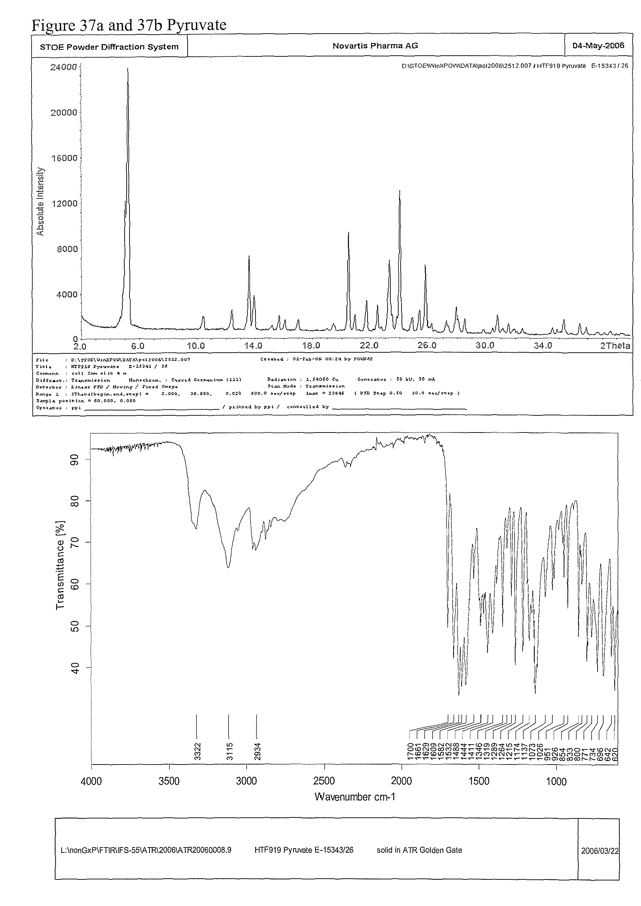

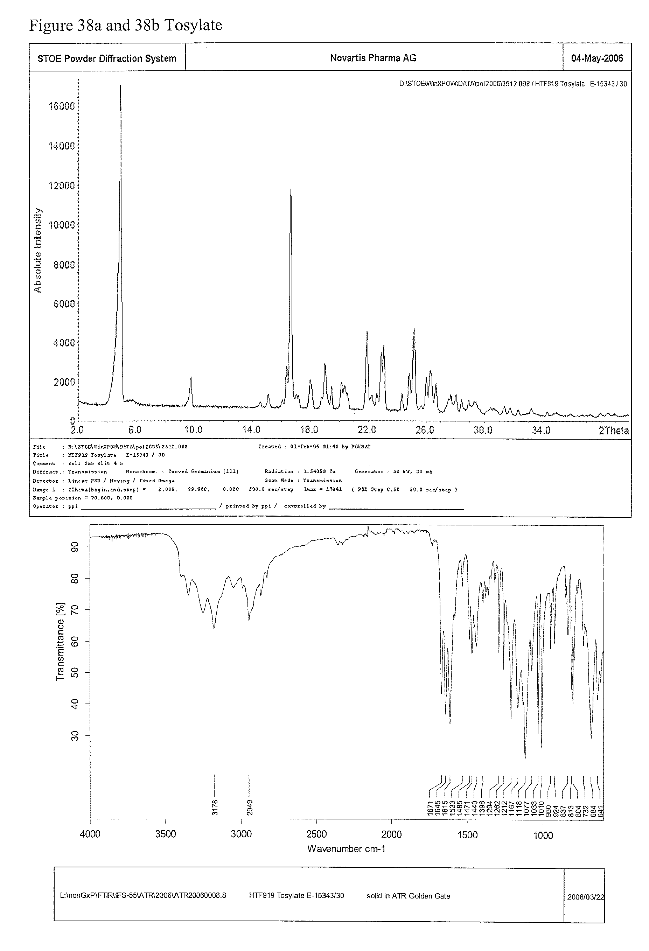

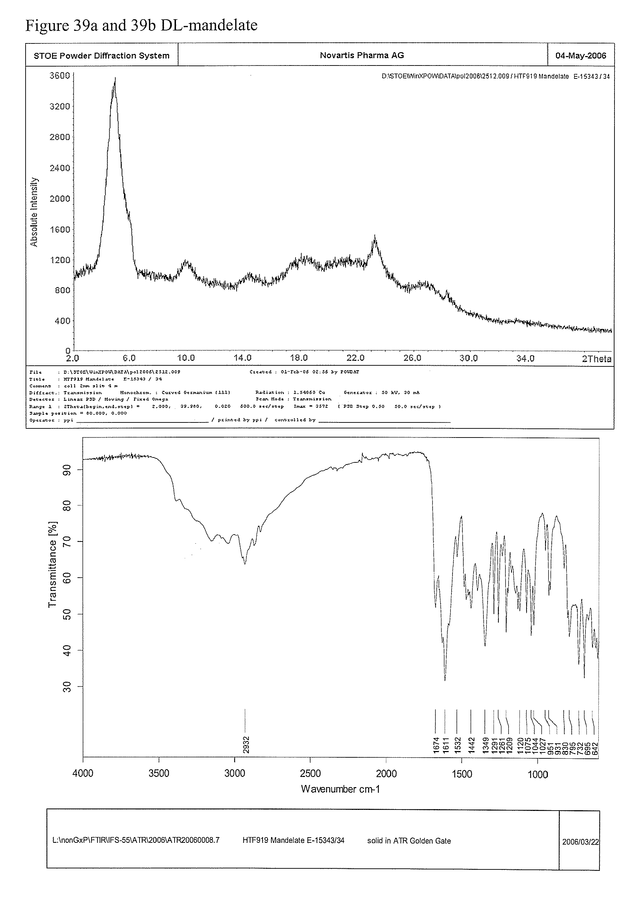

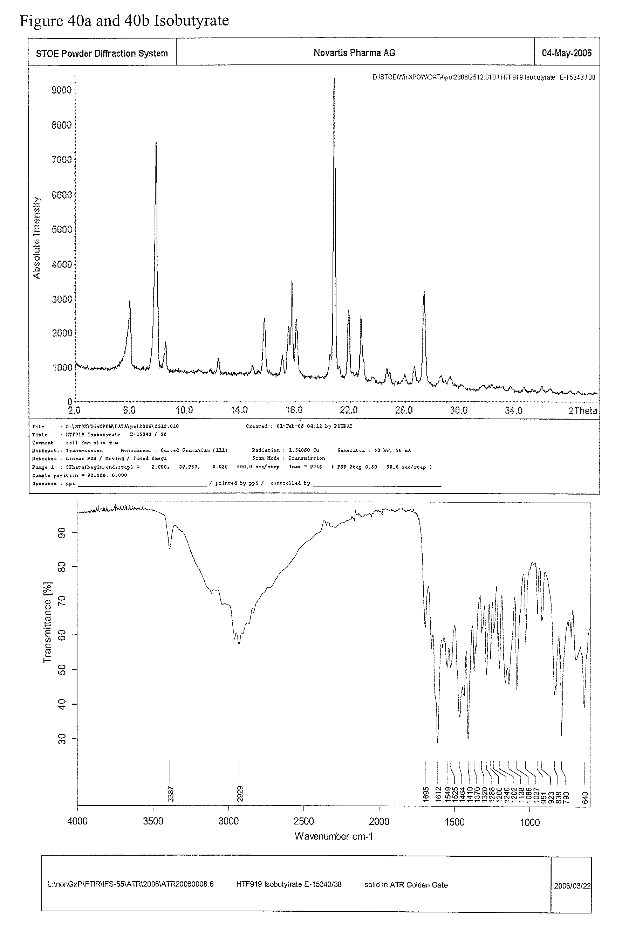

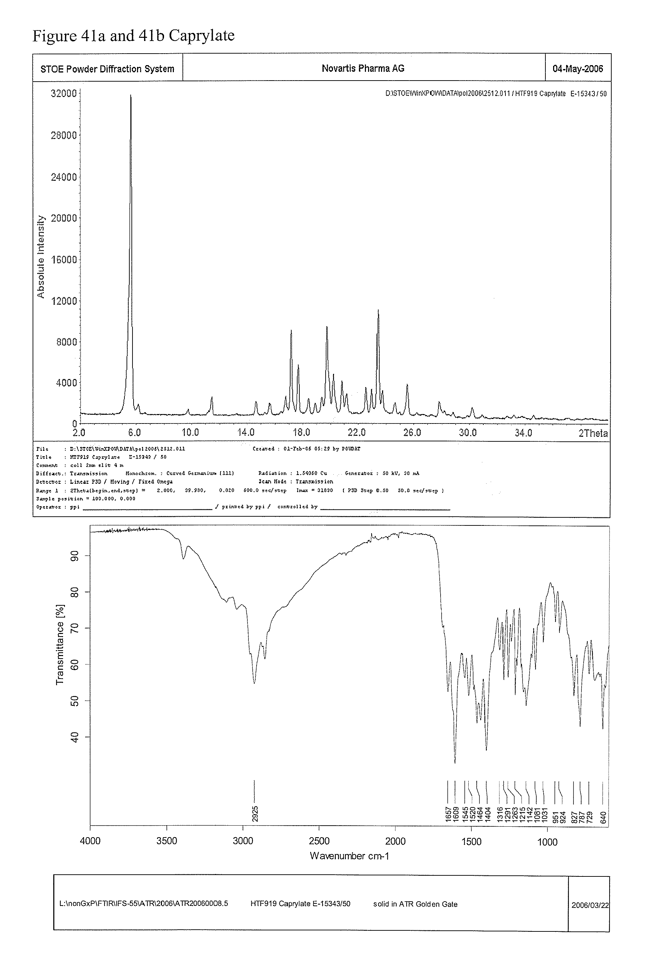

Figs. 1 -41 illustrate either or both of (a) an X-ray powder diffractogram of the stated salt of the invention as exemplified in the Examples below (lower X-axis: ° diffraction; right Y-axis: % signal) and (b) a Raman spectroscopy spectrum using a nujol mull between KBr plates.

The preparation of the salt forms of the present invention may be carried out, for example, as described in the non-limiting embodiments below.

### Examples

The following crystalline tegaserod salts (as shown by the X -ray powder diffraction diagram pattern) have been prepared at a multi gram scale for physico-chemical characterization.

### Example 1 glycolate

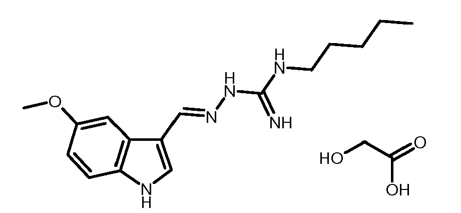

5.0 g base (16.59 mmoles) are dissolved in 35 ml ethanol at ca. 70°C. A solution of 1.33 g glycolic acid (17.42 mmoles) in 10 ml ethanol is added dropwise over 5 min. The dropping funnel is rinsed with 5 ml ethanol. The clear solution is allowed to cool and seeded at 40°C. Crystallization takes slowly place. The suspension is stirred over night at room temperature and filtered. The crystals are washed with 30 ml ethanol in 3 portions and dried first at 60°C and ca. 10 mbar for 17 h. and then at 80°C /ca. 10 mbar for 20 h. Yield: 5.57 g white powder (89%)
Elemental analysis:
Calc.: 57.28 % C; 7.21 % H; 18.55 % N; 16.96 % O
Found: 56.85 % C; 6.99 % H; 18.49 % N; 16.95 % O

### Example 2 DL-lactate

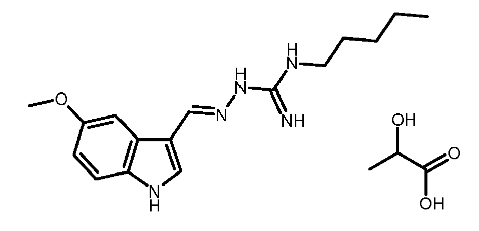

A suspension of 5.0 g base (16.59 mmoles) in 25 ml isopropanol is heated to 70°C. A solution of 2.37 g DL-lactic acid 90% (23.67 mmoles) in 7 ml isopropanol is added dropwise at constant flow rate over 5 min. The resulting solution is allowed to cool and seeded at 40°C. The suspension is stirred for 17 hours at room temperature and then filtered. The filter cake is washed three times with 10 ml isopropanol and dried at 60°C / ca 10 mbar for 20 hours.
Yield: 5.23g white crystals (80.5%)
Elemental analysis:
Calc.: 58.30 % C; 7.47 % H; 17.89 % N; 16.35 % O
Found: 58.06 % C; 7.43 % H; 17.58 % N; 16.69 % O
Water assay: 0.19% m/m

### Example 3 L-lactate

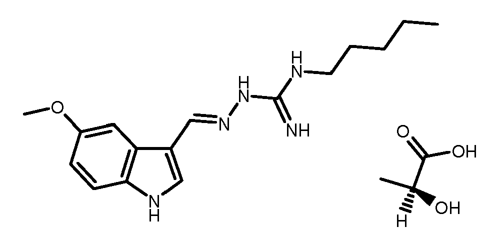

A suspension of 5.0 g base (16.59 mmoles) in 25 ml isopropanol is heated to 70°C. A solution of 3.93 g L-lactic acid 40% (17.45 mmoles) in 4 ml isopropanol is added dropwise over 5 min. The dropping funnel is rinsed with 1 ml isopropanol and the clear solution is allowed to cool. Crystallization occurs after seeding at 37°C. The suspension is stirred for 17 hours at room temperature and then filtered. The solid is washed three times with 10 ml isopropanol and dried at 60°C / ca 10 mbar for 20 hours.
Yield: 6.02 g white powder (92.7 %)
Elemental analysis:
Calc.: 58.30 % C; 7.47 % H; 17.89 % N; 16.35% O
Found: 58.02 % C; 7.31 % H; 17.74 % N; 16.46 % O
Water assay: 0.20 % m/m

### Example 4 acetate

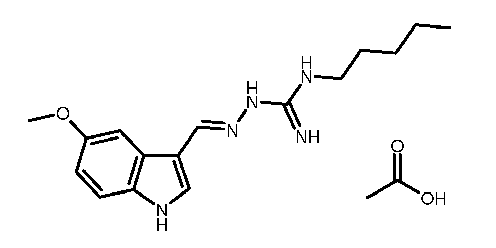

4.0 g base (13.27 mmoles) are dissolved in 16 ml acetic acid at 45°C. The solution is cooled to 25°C and 18 ml water are added over 10 min. The resulting suspension is stirred over night at room temperature. The solid is filtered by suction and washed successively with 10 ml acetic acid / water 1/1 and 16 ml water. The crystals are dried at 60°C and ca. 10 mbar for 20 hours.
Yield: 3.65 g white powder (76.0 %)
Elemental analysis:
Calc.: 59.82 % C; 7.53 % H; 19.38 % N; 13.28 % O
Found: 59.69 % C; 7.47 % H; 19.40 % N; 13.22 % O
Water assay: 0.11 % m/m

### Example 5 hydrogencitrate monohydrate

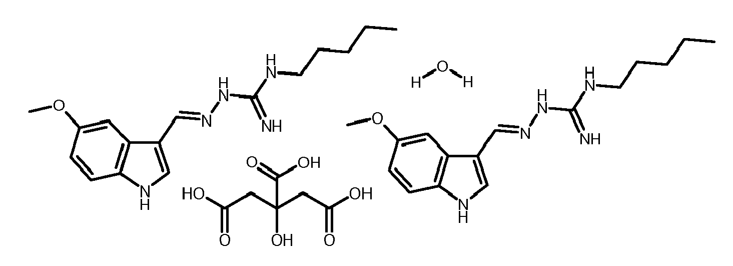

A suspension of 4.0 g base (13.27 mmoles) und 2.55 g citric acid (13.27 mmoles) in 36 ml ethanol is heated to ca. 75°C. 8 ml water are added dropwise over 10 min. The resulting solution is allowed to cool. Crystallization takes spontaneously place at 40°C. The slurry becomes rapidly too thick and is diluted with 20 ml ethanol /water = 82/18. After stirring over night at room temperature the salt is collected through filtration and washed with 15 ml ethanol /water = 82/18. A re-slurry wasch with 20 ml acetone is then performed and the solid is dried at 80°C and ca. 10 mbar for 20 h.
Yield: 4.24 g white powder (64.7%)
Elemental analysis:
Calc.: 56.15 % C; 6.94 % H; 17.23 % N; 19.68 % O
Found: 55.75 % C; 6.81 % H; 17.10 % N; 20.30 % O
Water assay (Karl Fischer Titration): 1.84 % m/m
Assay citric acid (ion-chromatography): 20.8 % m/m

### Example 6 sulfate monohydrate

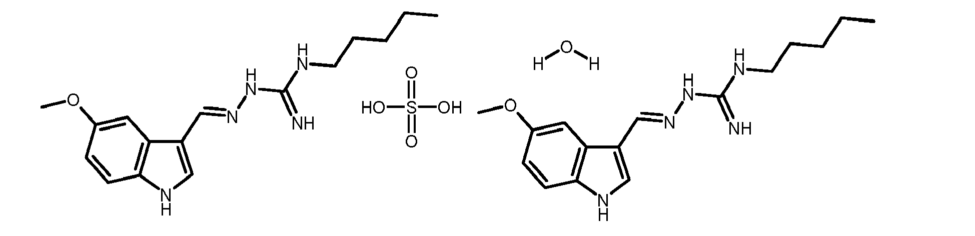

4.0 g base (13.27 mmoles) are dissolved in 20 ml methanol at ca. 60 °C. A solution of 1.33 g sulfuric acid 98% (13.27 mmoles) in 20 ml water is added dropwise over ca. 10 min. The resulting suspension is allowed to cool, stirred over night at r.t. and filtered after 18 h. The filter cake is washed with 11 ml methanol / water =1/1 and dried at 80°C and ca. 10 mbar for 20 h.
Yield: 4.39 g white powder (82.8%)
Elemental analysis:
Calc.: 53.47 % C; 7.01 % H; 19.48 % N; 15.58 % O; 4.46 % S
Found: 53.30 % C; 6.99 % H; 19.50 % N; 15.70 % O; 4.42 % S
Water assay: 2.67 % m/m

### Example 7 hydrogenmalate

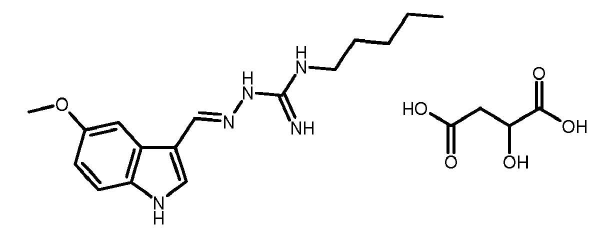

A suspension of 3.01 g base (10 mmoles) and 1.34 g (-) L-malic acid (10 mmoles) in 35 ml iosopropanol is heated to 75°C. The resulting clear solution is allowed to cool and seeded at 40°C. Crystallization takes place. The suspension is stirred for 20 h at room temperature. After filtration the salt is washed with 10 ml isopropanol and dried at 40°C and ca. 10 mbar for 20 h. Yield: 4.19 g white powder (96.3 %)
Elemental analysis:
Calc.: 55.16% C; 6.71% H; 16.08% N; 22.04% O
Found: 54.42% C; 7.09% H; 15.36% N; 22.85% O

### Example 8 malate

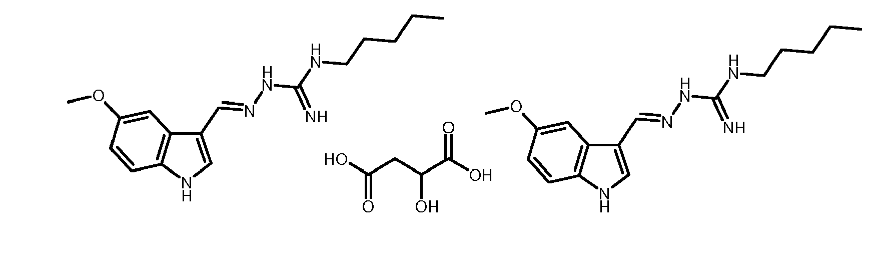

A suspension of 3.0 g base (9.95 mmoles) in 30 ml ethanol is heated to 60°C. 0.67 g (-) L-malic acid (4.98 mmoles) are added. The resulting solution is allowed to cool. Crystallization takes place after seeding at 40°C. The slurry is stirred at room temperature over night and filtered. The salt is washed with 10 ml ethanol. Then a re-slurry wash with 20 ml acetone is performed. The solid is dried at 80°C and ca. 10 mbar for 20 h. Yield: 3.60 g white powder (97.6 %)
Elemental analysis:
Calc.: 58.68 % C; 7.11 % H; 19.01 % N; 15.20% O
Found: 58.49 % C; 6.98 % H; 18.89 % N; 15.54 % O

### Example 9 glucuronate

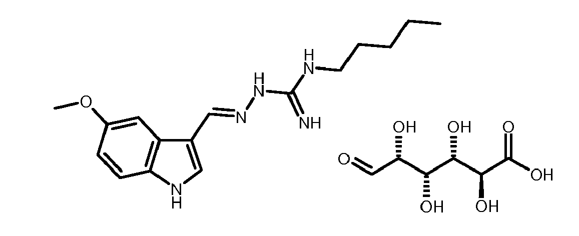

3.01 g base (10 mmoles) are dissolved in 20 ml methanol at 25°C. The mixture is heated at 55°C and a solution of 2.13 g D-glucuronic acid (11 mmoles) in 20 ml water is added over 10 min. The solution is seeded at 50°C and crystallization takes rapidly place. The slurry becomes rapidly too thick. The suspension is gradually diluted with 20 ml methanol / water = 1 / 1 during cooling and stirred over night at room temperature. The crystals are collected after filtration and washing with 20 ml methanol / water = 1 /1. Then a re-slurry wash with 20 ml acetone is performed. The solid is dried at 80°C and ca. 10 mbar for 20 Yield: 4.68 g white powder (94.7 %)
Elemental analysis:
Calc.: 53.32 % C; 6.71 % H; 14.13 % N; 25.83 % O
Found: 53.17 % C; 6.61 % H; 14.05 % N; 26.01 % O
Water assay: 0.19 % m/m

### Example 10 salicylate

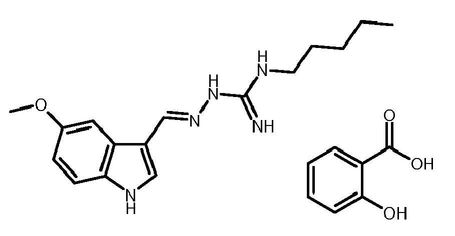

3.01 g base (10 mmoles) and 1.38 g salicylic acid (10 mmoles) are suspended in 30 ml isopropanol and the mixture is heated to 70°C. The resulting clear solution is allowed to cool and seeded at 50°C. The suspension is stirred over night at r.t. and filtered by suction. The filter cake is washed with 10 ml isopropanol and dried first for 2 h at 50°C and ca. 10 mbar and then for 20 h at 80°C and ca. 10 mbar.
Yield: 4.15 g white powder (94.5 %)
Elemental analysis:
Calc.: 62.85 % C; 6.65 % H; 15.93 % N; 14.56 % O
Found: 62.75 % C; 6.70 % H; 15.81 % N; 14.70 % O

### Example 11 gentisate

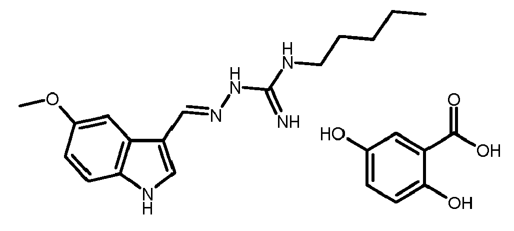

3.01 g base (10 mmoles) and 1.54 g 2.5-dihydrobenzoic acid acid (10 mmoles) are suspended in 30 ml ethanol and the mixture is heated to 60°C. The resulting clear solution is allowed to cool and seeded at 40°C. The suspension is stirred over night at r.t. and filtered by suction. The filter cake is washed with 10 ml ethanol and dried first for 2 h at 50°C and ca. 10 mbar and then for 20 h at 80°C and ca. 10 mbar.
Yield: 3.34 g white powder (73.4 %)
Elemental analysis:
Calc.: 60.65 % C; 6.42 % H; 15.37 % N; 17.56 % O
Found: 60.54 % C; 6.41 % H; 15.31 % N; 17.55 % O

### Example 12 hippurate

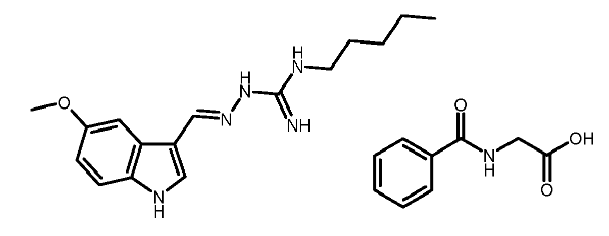

A suspension of 3.0 g base (9.95 mmoles) in 30 ml methanol is heated to 55°C. 1.84 g hippuric acid (9.95 mmoles) are added. The resulting solution is allowed to cool. Crystallization takes rapidly place after seeding at 40°C. The suspension becomes too thick and is diluted with 10 ml methanol. The slurry is stirred at room temperature over night and filtered. The salt is washed with 27 ml methanol in three portions. The solid is dried at 80°C and ca. 10 mbar for 20 h. Yield: 4.25 g white powder (88.8 %)
Elemental analysis:
Calc.: 62.48 % C; 6.71 % H; 17.49 % N; 13.32 % O
Found: 62.14 % C; 6.63 % H; 17.24 % N; 13.76 % O

### Example 13 succinate

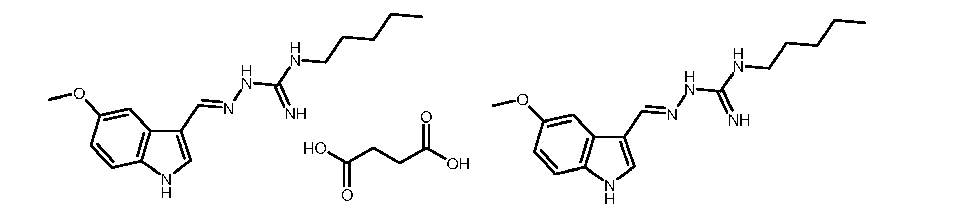

A suspension of 3.0 g base (9.95 mmoles) in 60 ml ethanol is heated to 70°C. 1.22 g succinic acid (10.33 mmoles) are added. The mixture is stirred for 10 min. at 70°C and the resulting solution is allowed to cool. Crystallization takes place after seeding at 50°C. The suspension is stirred at room temperature over night and filtered. The salt is washed with 30 ml ethanol in three portions. The solid is dried at 50°C and ca. 10 mbar for 20 h. Yield: 3.75 g white powder (98.9 %)
Elemental analysis:
Calc.: 59.98 % C; 7.27 % H; 19.43 % N; 13.32 % O
Found: 59.94 % C; 7.29 % H; 19.29 % N; 13.44 % O

### Example 14 fumarate

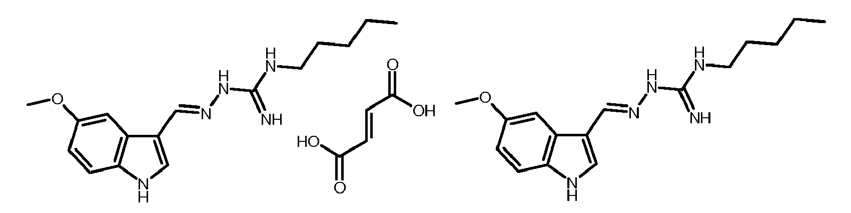

3.0 g base (9.95 mmoles) are dissolved in 60 ml ethanol at 70°C. 1.19 g fumaric acid (10.25 mmoles) are added. The solution is further stirred at 70°C. The salt crystallizes spontaneously after ca 10 min. The suspension is allowed to cool and stirred at 25°C for 20 h. The solid is collected after filtration and washing with 30 ml ethanol and dried for 20 h at 80°C and ca. 10 mbar.
Yield: 3.48 g white powder (97.2%)
Elemental analysis:
Calc.: 60.15 % C; 7.01 % H; 19.48 % N; 13.35 % O
Found: 60.13 % C; 6.99 % H; 19.35 % N; 13.44 % O

### Example 15 adipate

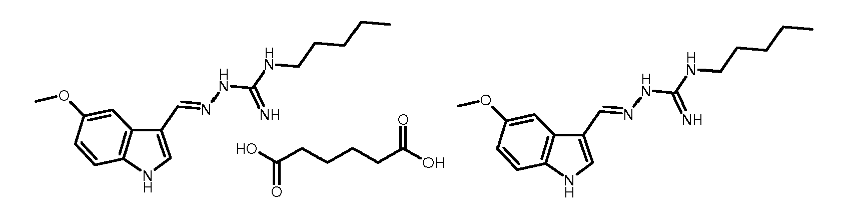

3.0 g base (9.95 mmoles) are dissolved in 60 ml methanol at 60°C. 1.46 g adipic acid (9,95 mmoles) are added. The resulting solution is allowed to cool and seeded at 40°C. The suspension is stirred at 25°C for 20 h and filtered. The crystals are washed with 30 ml methanol and dried for 20 h at 80°C and ca. 10 mbar.
Yield: 3.16 g white powder (84.7%)
Elemental analysis:
Calc.: 60.94 % C; 7.54 % H; 18.70 % N; 12.82 % O
Found: 60.63 % C; 7.60 % H; 18.70 % N; 13.08 % O

### Example 16 thiocyanate

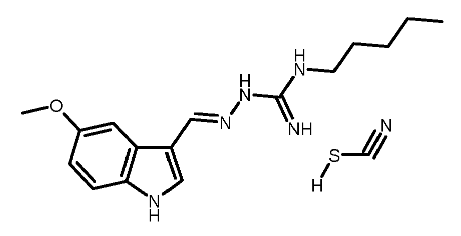

A suspension of 3.0 g base (9.95 mmoles) and 0.77 g ammonium rhodanide (10.1 mmoles) in 60 ml 3-methyl-1-butanol is heated at 70°C for 2 hours, until the gas evolution has stopped. The resulting solution is allowed to cool and is seeded at 30°C. The suspension is stirred at 25°C for 20 h and filtered. The solid is washed with 30 ml 3-methyl-1-butanol / tert. butyl methyl ether = 1 / 1 and dried for 20 h at 50°C and ca. 10 mbar.
Yield: 2.73 g white powder (76.2%)
Elemental analysis:
Calc.: 56.64 % C; 6.71 % H; 23.31 % N; 8.90 % S; 4.44 % O
Found: 56.84 % C; 6.74 % H; 23.27 % N; 8.69 % S; 4.63 % O

### Example 17 aspartate

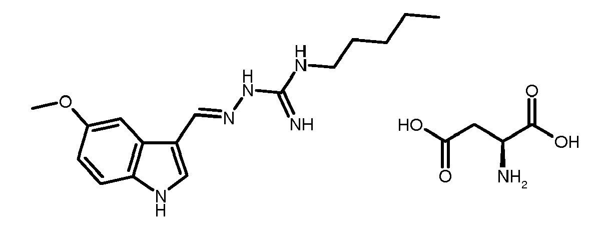

3.0 g base (9.95 mmoles) are dissolved in 120 ml methanol at 60°C. 1.33 g L-aspartic acid (10 mmoles) are added. The suspension is stirred for 10 min. at 60-65 °C The cloudy solution is then filtered. The filtrate is allowed to cool and seeded at 35°C. Crystallization takes slowly place. The suspension is stirred at 25°C for 20 h and filtered. The crystals are washed with 30 ml methanol and dried first for 2 h at 50°C /ca. 10 mbar and then for 20 h at 80°C and ca. 10 mbar.
Yield: 3.28 g white powder (75.7%)
Elemental analysis:
Calc.: 55.29 % C; 6.96 % H; 19.34 % N; 18.41 % O
Found: 55.19 % C; 6.85 % H; 19.40 % N; 18.60 % O

### Example 18 hydrogenmalonate

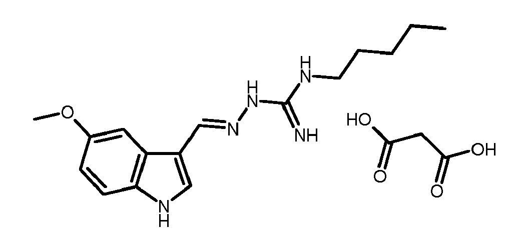

3.01 g base (10 mmoles) are dissolved in 60 ml methanol at 63°C. 1.05 g malonic acid (10.1 mmoles) are added. The resulting solution is allowed to cool and crystallization takes spontaneously place at ca. 58 °C. The suspension is stirred at 25°C for 20 h and filtered. The crystals are washed with 30 ml methanol and dried first for 2 h at 50°C /ca. 10 mbar and then for 20 h at 80°C and ca. 10 mbar.
Yield: 3.67 g white powder (90.5%)
Elemental analysis:
Calc.: 56.28 % C; 6.71 % H; 17.27 % N; 19.73 % O
Found: 56.18 % C; 6.61 % H; 17.28 % N; 19.85 % O

### Example 19 galactarate monohydrate

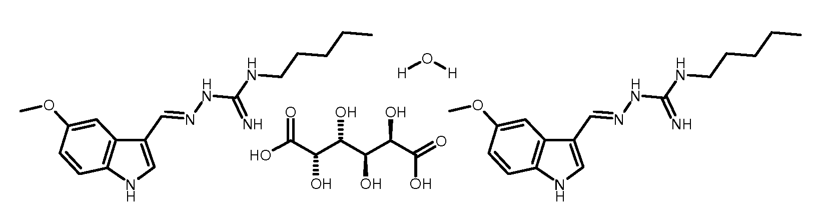

3.01 g base (10 mmoles) are dissolved in 60 ml methanol at 60°C. 1.05 g galactaric acid 97 % (~5 mmoles) are added. The suspension is stirred for 10 min. at 60°C. The resulting cloudy solution is filtered The clear filtrate is allowed to cool and seeded at 40°C. Crystallization takes rapidly place. The suspension is stirred at 25°C for 20 h and filtered. The crystals are washed with 15 ml methanol and dried first for 2 h at 50°C /ca. 10 mbar and then for 20 h at 80°C and ca. 10 mbar.
Yield: 3.77 g white powder (92.6 %)
Elemental analysis:
Calc.: 54.93 % C; 7.04 % H; 16.86 % N; 21.18 % O
Found: 54.30 % C; 7.16 % H; 16.74 % N; 21.40 % O
Water assay: 2.28 % m/m

### Example 20 oxoglutarate

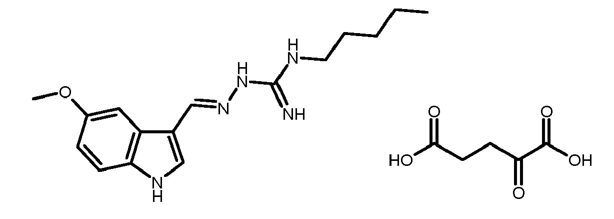

3.01 g base (10 mmoles) are dissolved in 90 ml isopropanol at 60°C. 1.46 g α-oxo-glutaric acid (10 mmoles) are added. The resulting solution is allowed to cool. Crystallization takes spontaneously place during cooling. The suspension is stirred at 25°C for 20 h and filtered. The crystals are washed with 30 ml isopropanol and dried first for 2 h at 50°C / ca. 10 mbar and then for 20 h at 80°C and ca. 10 mbar.
Yield: 4.40 g white powder (98.4 %)
Elemental analysis:
Calc.: 56.37 % C; 6.53 % H; 15.65 % N; 21.45 % O
Found: 56.41 % C; 6.63 % H; 15.72 % N; 21.40 % O

### Example 21 pidolate

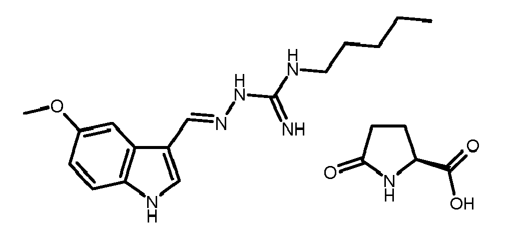

3.01 g base (10 mmoles) are dissolved in 90 ml acetone at 55°C. 1.29 g L-pyroglutamic acid (10 mmoles) are added. The resulting solution is allowed to cool. Crystallization takes place after seeding at 43 °C. The suspension is stirred at 25°C for 20 h and filtered. The crystals are washed with 30 ml acetone and dried first for 2 h at 50°C / ca. 10 mbar and then for 20 h at 80°C and ca. 10 mbar.
Yield: 4.23 g white powder (98.3 %)
Elemental analysis:
Calc.: 58.59 % C; 7.02 % H; 19.52 % N; 14.87 % O
Found: 58.40 % C; 7.22 % H; 19.44 % N; 15.17 % O

### Example 22 hydrogenglutarate

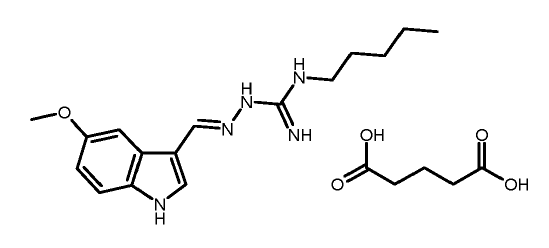

3.01 g base (10 mmoles) are dissolved in 45 ml ethanol at 65°C. 1.32 g glutaric acid (10 mmoles) are added. The resulting solution is allowed to cool. Crystallization takes place after seeding at 42 °C. The suspension is stirred at 25°C for 20 h and filtered. The crystals are washed with 15 ml ethanol and dried first for 2 h at 50°C / ca. 10 mbar and then for 20 h at 80°C and ca. 10 mbar. Yield: 3.85 g white powder (88.9 %)
Elemental analysis:
Calc.: 58.18%C;7.21 % H; 16.16 % N; 18.45 % O
Found: 58.21 % C; 7.25 % H; 16.16 % N; 18.44 % O

### Example 23 benzoate

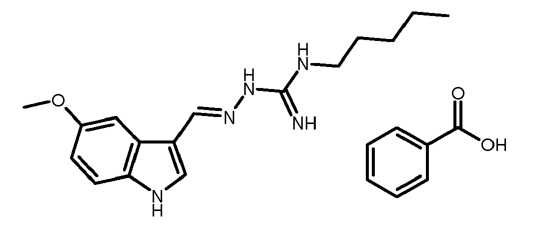

3.01 g base (10 mmoles) are dissolved in 36 ml ethanol at 65°C. 1.22 g benzoic acid (10 mmoles) are added. The resulting solution is allowed to cool. Crystallization takes place after seeding at ca. 40 °C. The suspension is stirred at 25°C for 20 h and filtered The crystals are washed with 15 ml ethanol and dried first for 2 h at 50°C / ca. 10 mbar and then for 20 h at 80°C and ca. 10 mbar. Yield: 4.12 g white powder (97.4 %)
Elemental analysis:
Calc.: 65.23 % C; 6.90 % H; 16.54 % N; 11.33 % O
Found: 65.16 % C; 7.08 % H; 16.51 % N; 11.36 % O

### Example 24 oxalate

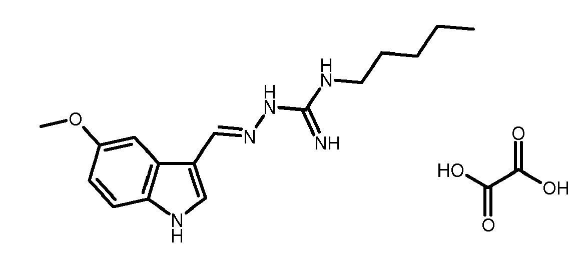

3.01 g base (10 mmoles) are dissolved in 60 ml ethanol at 65°C. 0.90 g oxalic acid (10 mmoles) are added.. The resulting cloudy solution is filtered. The clear filtrate is allowed to cool. Crystallization takes rapidly and spontaneously place. The suspension is stirred at 25°C for 20 h and filtered. The crystals are washed with 15 ml ethanol and dried first for 2 h at 50°C / ca. 10 mbar and then for 20 h at 80°C and ca. 10 mbar.
Yield: 3.68 g white powder (94.1 %)
Elemental analysis:
Calc.: 55.23 % C; 6.44 % H; 17.89 % N; 20.44 % O
Found: 54.74 % C; 6.29 % H; 17.80 % N; 20.79 % O

### Example 25 esylate

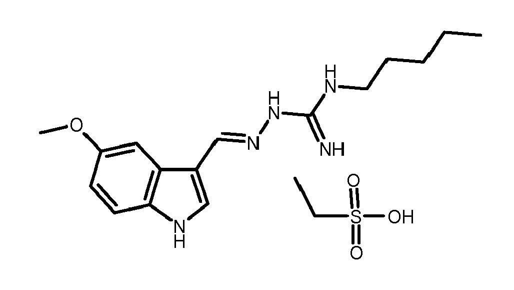

A suspension of 3.01 g base (10 mmoles) in 36 ml acetone is heated to 50°C. 1.16 g ethanesulfonic acid (10.5 mmoles) are added. The resulting clear solution is allowed to cool. Crystallization takes spontaneously place at ca. 45 °C. The suspension is stirred at 25°C for 20 h and filtered. The solid is washed with 15 ml acetone and dried first for 2 h at 50°C / ca. 10 mbar and then for 20 h at 80°C and ca. 10 mbar.
Yield: 3.93 g whitish powder (95.6 %)
Elemental analysis:
Calc.: 52.54 % C; 7.10 % H; 17.02 % N; 7.79 % S; 15.55 % O
Found: 52.65 % C; 7.01 % H; 17.07 % N; 7.70 % S; 15.59 % O

### Example 26 mesylate

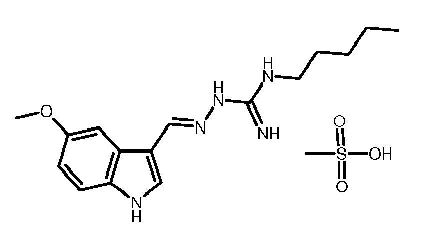

A suspension of 3.01 g base (10 mmoles) in 36 ml acetone is heated to 50°C. 0.97 g methanesulfonic acid (10.09 mmoles) are added. The resulting clear solution is stirred at 50-55°C. Crystallization takes spontaneously place after ca. 5 min. The suspension is allowed to cool and stirred at 25°C for 20 h. The salt is filtered by suction. The filter cake is washed with 15 ml acetone and dried first for 2 h at 50°C / ca. 10 mbar and then for 20 h at 80°C and ca. 10 mbar.
Yield: 3.76 g yellowish powder (94.7 %)
Elemental analysis:
Calc.: 51.37 % C; 6.85 % H; 17.62 % N; 8.07 % S; 16.10 % O
Found: 51.28 % C; 6.82 % H; 17.62 % N; 8.19 % S; 16.13 % O

### Example 27 napadisilate

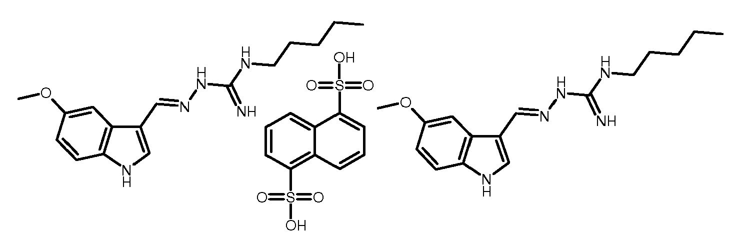

A suspension of 3.01 g base (10 mmoles) in 45 ml acetone is heated to 50°C. A solution of 2.88 g naphthalene-1,5-disulfonic acid (10 mmoles) in 6 ml water is added drop-wise. The resulting clear solution is allowed to cool. Crystallization takes rapidly place during cooling. The suspension is stirred at 25°C for 20 h. The salt is filtered by suction. The filter cake is washed with 12 ml acetone / water 1 / 1. A re-slurry wash with 12 ml acetone is performed and the solid is dried first for 2 h at 50°C / ca. 10 mbar and then for 20 h at 80°C and ca. 10 mbar.
Yield: 2.51 g whitish powder (56.4 %) Assay water: 0.29 % m/m
Elemental analysis:
Calc.: 56.61 % C; 6.11 % H; 15.72 % N; 7.20 % S; 14.36 % O
Found: 56.55 % C; 6.06 % H; 15.73 % N; 7.18 % S; 14.44 % O

### Example 28 formate

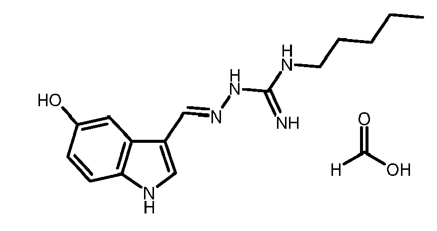

3.01 g base (10 mmoles) are dissolved in 45 ml ethanol at 65°C. 0.47 g formic acid (10.2 mmoles) are added. The solution is allowed to cool. Crystallization begins at ca. 48 °C. The suspension is stirred at 25°C for 20 h and filtered. The crystals are washed with 20 ml ethanol and dried first for 2 h at 50°C / ca. 10 mbar and then for 20 h at 80°C and ca. 10 mbar.
Yield: 3.14 g white powder (90.5 %)
Elemental analysis:
Calc.: 58.77 % C; 7.25 % H; 20.16 % N; 13.82 % O
Found: 58.85 % C; 7.40 % H; 20.34 % N; 13.76 % O

### Example 29 quadrate

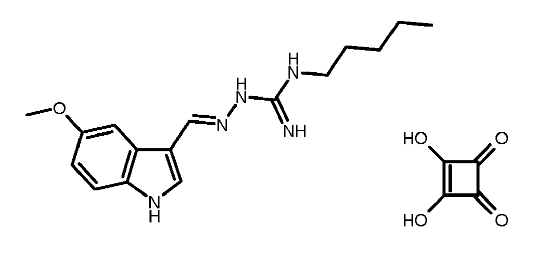

1.18 g 3,4-dihydroxy-3-cyclobutene-1,2-dione (squaric acid) (10.3 mmoles) are added at 60°C to a solution of 3.03 g base (10 mmoles) in 60 ml methanol. The suspension is diluted with 15 ml methanol and stirred for 10 min. at 65 °C. The resulting solution is allowed to cool. Crystallization takes place after seeding at 45°C. The suspension is stirred at 25°C for 20 h and filtered. The crystals are washed with 20 ml methanol and dried first for 2 h at 50°C / ca. 10 mbar and then for 20 h at 80°C and ca. 10 mbar. Yield: 3.54 g white powder (85.3 %)
Elemental analysis:
Calc.: 57.82 % C; 6.07 % H; 16.86 % N; 19.26 % O
Found: 57.90 % C; 6.04 % H; 16.88 % N; 19.36 % O

### Example 30 propionate

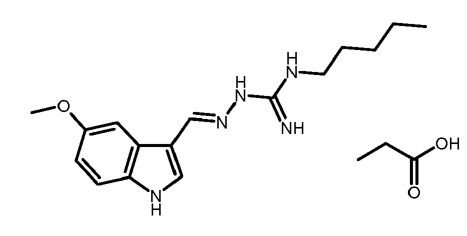

0.75 g propionic acid (10.1 mmoles) are added at 60°C to a solution of 3.01 g base (10mmoles) in 54 ml methanol. The clear solution is allowed to cool. Crystallization takes place after seeding at 45°C. The suspension is stirred at 25°C for 20 h and filtered. The crystals are washed with 18 ml methanol and dried first for 2 h at 50°C /ca. 10 mbar and then for 20 h at 80°C and ca. 10 mbar.
Yield: 3.20 g white powder (85.3 %)
Elemental analysis:
Calc.: 60.78 % C; 7.78 % H; 18.65 % N; 12.78 % O
Found: 60.74 % C; 7.73 % H; 18.73 % N; 12.87 % O

### Example 31 dichloroacetate

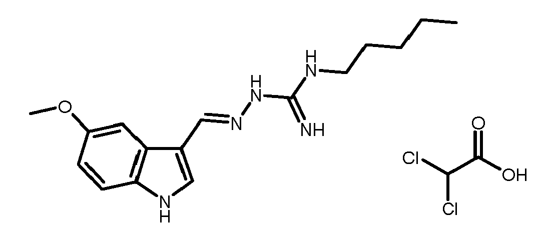

A suspension of 3.01g base (10 mmoles) in 54 ml isopropanol is heated at 70°C. 1.31 g dichloroacetic acid (10.1 mmoles) are added. The solution is allowed to cool. Crystallization takes place after seeding at ca. 60 °C. The suspension is stirred at 25°C for 20 h and filtered. The crystals are washed with ca. 15 ml isopropanol and dried first for 2 h at 50°C / ca. 10 mbar and then for 20 h at 80°C and ca. 10 mbar.
Yield: 4.13 g white powder (96.0 %)
Elemental analysis:
Calc.: 50.24 % C; 5.86 % H; 16.27 % N; 16.48 Cl; 11.15% O
Found: 50 03 % C; 5.69 % H; 16.56 % N; 16.44 Cl; 11.26 % O

### Example 32 acetamidobenzoate

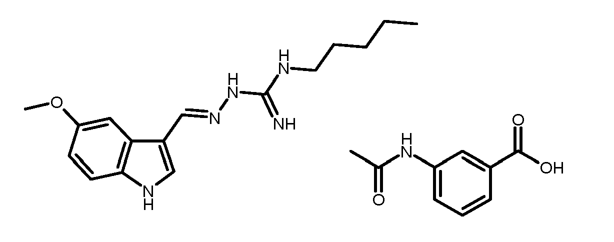

1.83 g 4-acetamidobenzoic acid (10.1 mmoles) are added at 65°C to a solution of 3.01 g base (10mmoles) in 45 ml ethanol. The clear solution is allowed to cool. Crystallization takes place after seeding at 45°C. The suspension is stirred at 25°C for 20 h and filtered. The crystals are washed with 18 ml ethanol and dried first for 2 h at 50°C / ca. 10 mbar and then for 20 h at 80°C and ca. 10 mbar.
Yield: 4.49 g white powder (93.5 %)
Elemental analysis:
Calc.: 62.48 % C; 6.71 % H; 17.49 % N; 13.32 % O
Found: 62.36 % C; 6.73 % H; 17.50 % N; 13.46 % O

### Example 33 besilate

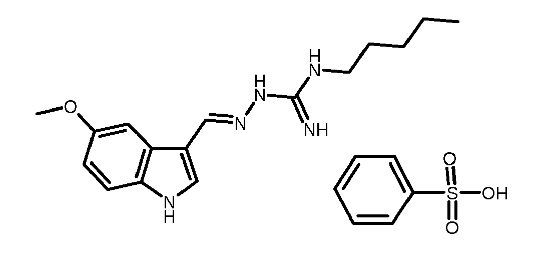

A suspension of 3.01 g base (10 mmoles) in 36 ml acetone is heated to 50°C. 1.62 g benzenesulfonic acid (10.2 mmoles) are added. The resulting clear solution is stirred at 50-55°C. Crystallization takes spontaneously place after ca. 5 min. The suspension is allowed to cool and is stirred at 25°C for 20 h and filtered. The solid is washed with 18 ml acetone and dried first for 2 h at 50°C / ca. 10 mbar and then for 20 h at 80°C and ca. 10 mbar.
Yield: 4.39 g whitish powder (95.6 %)
Elemental analysis:
Calc.: 57.50 % C; 6.36 % H; 15.24 % N; 6.98 % S; 13.93 % O
Found: 57.50 % C; 6.23 % H; 15.05 % N; 6.90 % S; 13.96 % O

### Example 34 pyruvate

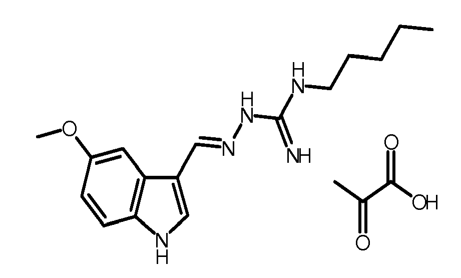

A suspension of 3.01 g base (10 mmoles) in 36 ml acetone is heated to 50°C. 0.90 g pyruvic acid (10.2 mmoles) are added. The resulting clear solution is stirred at 50-Reflux temperature. Crystallization takes spontaneously place after ca. 5 min. The suspension is allowed to cool and is stirred at 25°C for 20 h and filtered. The solid is washed with 15 ml acetone and dried first for 2 h at 50°C / ca. 10 mbar and then for 20 h at 80°C and ca. 10 mbar.
Yield: 3.76 g white powder (96.6 %)
Elemental analysis:
Calc.: 58.60 % C; 6.99 % H; 17.98 % N; 16.43 % O
Found: 58.44 % C; 6.88 % H; 17.51 % N; 16.74 % O

### Example 35 tosylate

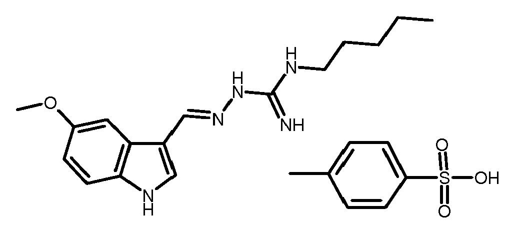

A suspension of 3.01 g base (10 mmoles) in 36 ml acetone is heated to 50°C. 1.90 g p-toluenesulfonic acid monohydrate (11 mmoles) are added. The resulting clear solution is allowed to cool. Crystallization takes place after seeding at 40 °C. The suspension becomes rapidly too thick and is gradually diluted with 54 ml acetone during cooling. The slurry is stirred at 25°C for 20 h and filtered. The crystals are washed with 15 ml acetone and dried first for 2 h at 50°C / ca. 10 mbar and then for 20 h at 80°C and ca. 10 mbar. Yield: 4.13 g white powder (87.3 %)
Elemental analysis:
Calc.: 58.33 % C; 6.60 % H; 14.79 % N; 6.77 % S; 13.51 % O
Found: 58.15 % C; 6.50 % H; 14.56 % N; 6.52 % S; 13.55 % O

### Example 36 isobutyrate

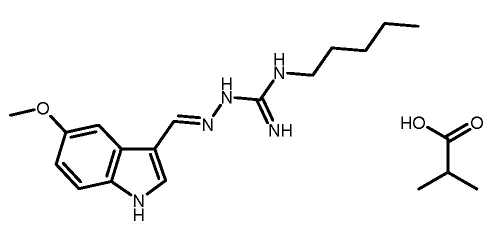

A suspension of 3.01g base (10 mmoles) in 30 ml isopropanol is heated at 65°C. 0.89 g isobutyric acid (10.1 mmoles) are added. The solution is allowed to cool. Crystallization takes place after seeding at ca. 60 °C. The suspension becomes rapidly too thick and is gradually diluted with 60 ml isopropanol during cooling. The slurry is stirred at 25°C for 20 h and filtered. The crystals are washed with 30 ml isopropanol and dried first for 2 h at 50°C / ca. 10 mbar and then for 20 h at 80°C and ca. 10 mbar.
Yield: 3.19 g white powder (82.0 %)
Elemental analysis:
Calc.: 61.67 % C; 8.02 % H; 17.98 % N; 12.32 % O
Found: 61.79 % C; 7.92 % H; 18.06 % N; 12.47 % O

### Example 37 caprylate

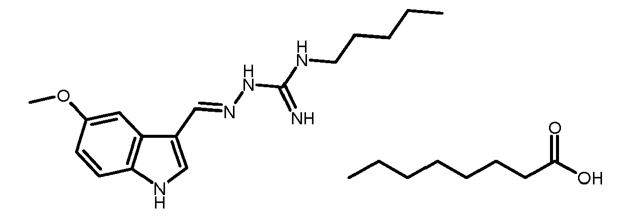

A suspension of 3.01g base (10 mmoles) in 45 ml isopropanol is heated at 70°C. 1.46 g caprylic acid (10.1 mmoles) are added. The solution is allowed to cool. Crystallization takes place after seeding at ca. 50 °C. The suspension becomes too thick and is gradually diluted with 15 ml isopropanol during cooling. The slurry is stirred at 25°C for 20 h and filtered. The crystals are washed with 12 ml isopropanol and dried first for 2 h at 50°C / ca. 10 mbar and then for 20 h at 80°C and ca. 10 mbar. Yield: 3.38 g white powder (75.9 %)
Elemental analysis:
Calc.: 64.69 % C; 8.82 % H; 15.72 % N; 10.77 % O
Found: 64.78 % C; 8.76 % H; 15.87 % N; 10.86 % O

### Example 38 nitrate

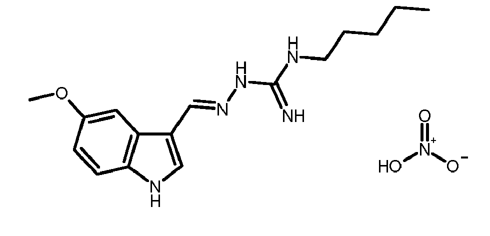

3.01 g base (10 mmoles) are dissolved in 30 ml methanol at room temperature. 0.64 g nitric acid fuming (~ 10 mmoles) are added dropwise. The resulting orange suspension is heated to 65°C and 15 ml water and 30 ml methanol are slowly added, until a clear solution is obtained. The solution is allowed to cool. Crystallization takes place after seeding at 40°C. The suspension is stirred at 25°C for 20 h and filtered. The crystals are washed with 20 ml methanol and dried first for 2 h at 50°C / ca. 10 mbar and then for 20 h at 80°C and ca. 10 mbar.
Yield: 2.73 g orange powder (75.0 %)
Elemental analysis:
Calc.: 52.74 % C; 6.64 % H; 23.06 % N; 17.56 % O
Found: 52.71 % C; 6.46 % H; 23.23 % N; 17.81 % O
Water assay < 0.3 % m/m

### Example 39 xinafoate

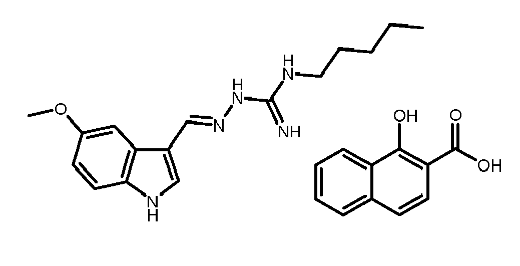

A suspension of 2.0 g base (6.63 mmoles) and 1.25 g 1-hydroxy-2-naphthoic acid (6.63 mmoles) in 30 ml isopropanol is heated at 70°C. The resulting clear solution is allowed to cool. Crystallization takes place after seeding at 30°C. The slurry is stirred at 25°C for 20 h and filtered. The crystals are washed with 20 ml isopropanol and dried for 20 h at 80°C and ca. 10 mbar. Yield: 2.69 g whitish powder (82.7%)
Elemental analysis:
Calc.: 66.24 % C; 6.38 % H; 14.31 % N; 13.07 % O
Found: 66.29 % C; 6.37 % H; 14.33 % N; 13.49 % O
Water assay: 0.29 % m/m

### Example 40 pamoate

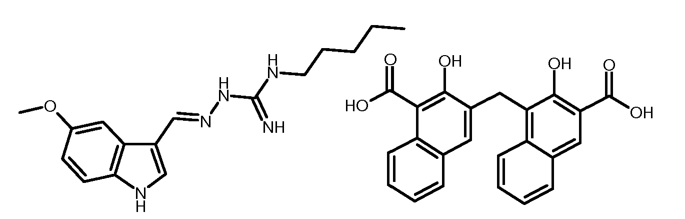

2.0 g base (6.63 mmoles) and 2.57 g pamoic acid (6.63 mmoles) are dissolved in 35 ml dimethylsulfoxide at 40°C. The solution is cooled to 25°C and 17 ml water are added dropwise over ca. 10 min. The cloudy solution containing some sticky solid material is heated to 70°C. The resulting suspension is then allowed to cool and is stirred for 5 h at room temperature. The salt is isolated via filtration and successively washed with 15 ml DMSO / water = 1 / 1 and with 15 ml DMSO. A re-slurry wash with 20 ml acetone is performed and the product is dried at 80°C and ca. 10 mbar for 20 h. Yield: 3.85 g yellowish powder (84.2 %)
Elemental analysis:
Calc.: 67.91 % C; 5.70 % H; 10.15 % N; 16.24 % O
Found: 67.71 % C; 5.69 % H; 10.12 % N; 16.37 % O
Water assay: 0.1 % m/m

### Preparation of the semi amorphous salts

### Example 41 D-gluconate hemihydrate

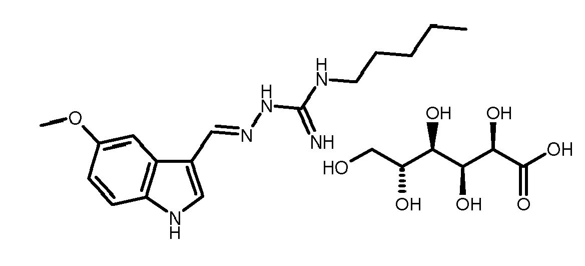

3.01 g base (10 mmoles) and 3.92 g D-gluconic acid (50 % solution in water) (10 mmoles) are suspended in 100 ml isopropanol and the mixture is heated to ca. 70°C. The resulting clear solution is allowed to cool. Precipitation of solid material begins slowly at ca. 50°C. The slurry is stirred over night at r.t. and filtered by suction. The filtration is very slow. The filter cake is washed with 20 ml isopropanol. A re-slurry wash with 20 ml heptane is performed and the solid is dried for 20 h at 40°C and ca. 10 mbar. Yield: 3.72 g white powder (74.8 %)
Elemental analysis:
Calc.: 53.11 % C; 7.09 % H; 14.08 % N; 25.73 % O
Found: 52.22 % C; 7.14 % H; 13.39 % N; 26.84 % O
Water assay: 1.56 % m/m

### Example 42 DL-mandelate

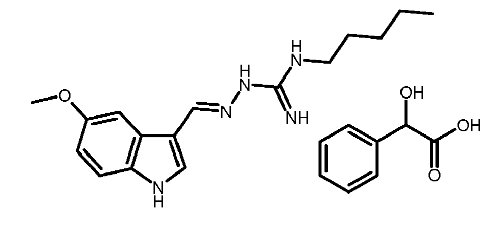

A suspension of 3.01 g base (10 mmoles) in 60 ml ethylacetate is heated at 70°C. 1.54 g DL-mandelic acid (10.1 mmoles) are added. The resulting solution is allowed to cool. Slow precipitation begins at ca. 40 °C. The slurry is stirred at 25°C for 20 h and filtered. The filter cake is washed with 30 ml ethylacetate and dried first for 2 h at 50°C / ca. 10 mbar and then for 20 h at 80°C and ca. 10 mbar.
Yield: 4.00 g yellowish powder (88.3 %)
Elemental analysis:
Calc.: 63.56 % C; 6.89 % H; 15.44 % N; 14.11 % O
Found: 63.39 % C; 6.89 % H; 15.20 % N; 14.16 % O

### Example 43 lactobionate

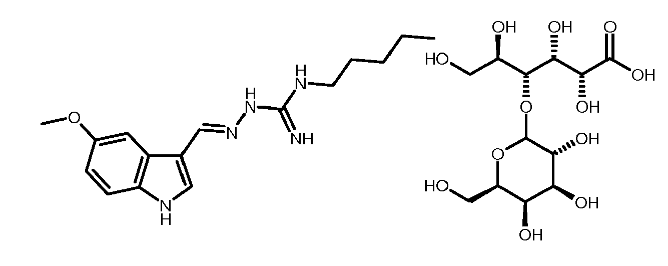

A suspension of 2.0 g base (6.63 mmoles) and 2.38 g lactobionic acid (6.63 mmoles)in 40 ml ethanol is heated at 75°C. 0.4 ml water are added. The resulting cloudy solution is filtered. The clear filtrate is allowed to cool. Precipitation begins at ca. 50 °C. Initially sticky, semi solid constituents are transformed in a homogeneous suspension during cooling. The slurry is stirred at 25°C for 20 h and filtered. Filtration is very fast. The filter cake is washed with 10 ml ethanol and dried for 20 h at 50°C and ca. 10 mbar.
Yield: 2.36 g white powder (53.8 %)
Elemental analysis:
Calc.: 50.98 % C; 6.88 % H; 10.62 % N; 31.53 % O
Found: 49.85 % C; 6.78 % H; 10.02 % N; 33.23 % O
Water assay: 1.28 % m/m

### Example 44: A 2 mg tablet formulation of a tablet of a salt of the invention may be prepared as described hereinafter

### a) Preparation of the granulated material:

### Premixing step:

1. 4.432 kg of salt of 3-(5-methoxy-1H-indol-3-yl-methylene)-N-pentylcarbazimidamide and 28.688 kg of lactose monohydrate are mixed with an intensive mixer (Colette Gral® 300 1 or Fielder® ; mixer speed setting : 1; chopper speed setting : 1) for approximately 1.5 minutes, or with a free fall mixer (Turbula®, Soneco® or Röhnrad®)
2. The pre-mixture from step 1 is then sieved (oscillating granulator, e. g., Frewitt® or Erweka®; mesh size: 0.8 millimetres).
3. The pre-mixture is divided into two portions of 16.560 kg.

### Preparation of the granulating solution

4. Approximately 40 kg of purified water are weighed out.
5. 3.600 kg of methylhydroxypropylcellulose 3 maps are added to the purified water from step 4 and this is stirred until dissolution.
6. 1.440 kg of poloxamer 188 are added to the solution from step 5 while stirring until dissolution.

### Granulating step:

7. 28.800 kg of crospovidone, 10.080 kg of lactose monohydrate and 4.320 kg of glyceryl monostearate are weighed out.
8. One portion of the premixture from step 3 is added to the excipients from step 7 and this is mixed with the intensive mixer, e.g., Colette Gral® 300 1 or Fielder® (mixer speed setting : 1; chopper speed setting : 1) for approximately 2 minutes.
9. The mixture from step 8 is wetted with the granulating solution from step 6 while mixing with the intensive mixer, e.g., Colette Gral® 300 I or Fielder® (mixer speed setting : 1; chopper speed setting : 0; pumping rate approximately : 4 kg/minute) for approximately 12 minutes.
10. Approximately 2 kg of purified water are weighed out.
11. The vessel from step 6 is rinsed with the purified water from step 10 and this is added to the mixture from step 9 while mixing.
12. The mass is granulated by mixing with the intensive mixer, Colette Gral® 300 1 or Fielder® (mixer speed setting : 1; chopper speed setting : 1) for approximately 2.5 minutes.

### Drying step

13. The granulate from step 12 is dried in a fluidised air bed drier (e. g., Glatt® or Aeromatic®) for approximately 65 minutes (inlet air temperature approximately 70°C) to reach the required loss on drying (LOD) for the tabletting mixture, i. e., until LOD - <4,4%.
14. The granulate sized by sieving (0.8 millimetres) with an oscillating sieve granulator, e.g., Frewitt® or Erweka®.
15. Steps 4 to 14 are repeated with the other portion of step 3.

### b) Preparation of the tabletting mixture

16. 8.640 kg of polyethylene glycol 4000 and 5.760 kg of glyceryl monostearate are sieved (oscillating granulator, e.g., Frewitt® or Erweka®; mesh size: 0.8 millimetres)
17. The ingredients from step 16 are added to the total mass of granulated material and this is mixed with a free fall mixer, e.g., Soneco® or Röhnrad®, for approximately 20 minutes (10 rpm) to obtain the desired final tabletting mixture.

### c) Compression step

18. The tabletting mixture from step 17 is pressed into tablets using compression pressures of 11, 14 or 17 KN on a rotary tabletting machine, e.g., Fette®, Korsh®, Kelian® or Coarty® (temperature < 20°C; M (relative humidity) < 40%)

### Example 5: Composition of a 2 mg tablet of a salt of 3-(5-methoxy-1H-indol-3-yl-methylene)-N-pentylcarbazimidamide)

| | |
|---|---|
| Salt | 2.77 |
| mg (2mg base) | |
| Polyplasdone XL USP/NF | 36.00 mg |
| Glyceryl monostearate USP/NF | 9.00 mg |
| Poloxalkol | 1.80 |
| mg | |
| Lactose 200 mesh | 30.53 mg |
| HPMC 3cPs | 4.50 mg |
| Polyethyleneglycol 4000 | 5.40 mg |
| Water adsorbed | 2.00 |
| mg | |
| Total | 92 |
| mg | |

Example 6: A 6 mg tablet formulation of a salt of the invention may be prepared by the manufacturing process described hereinafter.

### a) Preparation of the granulated material

### Preparation of the granulating solution

1. Approximately 40 kg of purified water are weighed out.
2. 3.600 kg of methylhydroxypropylcellulose 3 maps are added to the purified water from step 1 while stirring until dissolution.
3. 1.440 kg of poloxamer 188 are added to the solution from step 2 while stirring until dissolution (mixing tank under stirring).

### Granulating step

4. 4.787 kg of a salt of 3-(5-methoxy-1 H-indol-3-yl-methylene)-N-pentylcarbazimidamide and 28.800 kg of crospovidone, 21.853 kg of lactose monohydrate and 4.320 kg of glyceryl monostearate are weighed out.
5. The ingredients from step 4 are mixed with the intensive mixer, e.g., Colette Gral® 300 l or Fielder® (mixer speed setting: 1; chopper speed setting : 1) for approximately 2 minutes.
6. The mixture from step 5 is wetted with the granulating solution from step 3 while mixing with the intensive mixer, e.g., Colette Gral® 300 I or Fielder® (mixer speed setting: 1; chopper speed setting : 0; pumping rate approximately 4 kg/minute) for approximately 12 minutes.
7. Approximately 2 kg of purified water are weighed out.
8. The vessel from step 3 is rinsed with the purified water from step 7 and this is added to the mixture from step 6 while mixing.
9. The mass is granulated by mixing with the intensive mixer, e.g., Colette Gral® 300 I or Fielder® (mixer speed setting: 1; chopper speed setting : 1) for approximately 2.5 minutes.

### Drying step

10. The granulate from step 9 is dried in a fluidised air bed drier, e.g. , Glatt® or Aeromatic® for approximately 65 minutes (Inlet air temperature approximately 70°C) to reach the desired loss on drying (LOD) for the tabletting mixture, i. e., until LOD ≤4,4%.
11. The granulate sized by sieving (0.8 millimetres) with an oscillating sieve granulator (Frewitt® or Erweka®
12. Steps 1 to 11 are repeated.

### b) Preparation of the tabletting mixture

13. 8.640 kg of polyethylene glycol 4000 and 5.760 kg of glyceryl monostearate are sieved with an oscillating sieve granulator, e.g., Frewitt® or Erweka® (0.8 millimetres)
14. The ingredients from step 13 are added to the total mass of granulated material and this is mixed with a free fall mixer, e.g., Soneco® or Röhnrad®, for approximately 20 minutes (10 rpm) in the desired final tabletting mixture.

### c) Compression step

15. The tabletting mixture from step 14 is pressed into tablets using compression pressures of 13, 16 or 19 KN on a rotary tabletting machine, e.g., Fette®, Korsh®, Kelian® or Coarly® (temperature<200C, M (relative humidity) < 40 %).

### Example 7: Composition of a 6 mg tablet of a salt of 3-(5-methoxy-1 H-indol-3-yl-methylene)-N-pentylcarbazimidamide)

| | |
|---|---|
| Salt | 8.31 |
| mg (6mg base) | |
| Polyplasdone XL USP/NF | 50.00 mg |
| Glyceryl monostearate USP/NF | 12.50 mg |
| Poloxalkol | 2.50 |
| mg | |
| Lactose 200 mesh | 37.94 mg |
| HPMC 3cPs | 6.25 mg |
| Polyethyleneglycol 4000 | 7.50 mg |
| Water adsorbed | 3.00 |
| mg | |
| Total | 128 |
| mg | |

### EXAMPLE 8: Formulations of a salt of 3-(5-methoxy-1H-indol-3-yl-methylene)-N-pentylcarbazimidamide

A 6 mg tablet is prepared using roller compaction (see also Example 4 of WO 03/053432):

| Component | Quantity (125 mg tablet) % w/w |
|---|---|
| salt | 6.65 |
| Lactose spray dried | 76.85 |
| Crospovidone | 10.00 |
| Aérosil | 0.50 |
| glyceryl behenate | 6.00 |

Compositions are prepared by mixing the tegaserod salt, lactose, crospovidone, aérosil and glyceryl behenate. This mixture is compacted by roller compaction and milled. Tablets are formed by compression.

### EXAMPLE 9: Further compositions (direct compression)

Further compositions may be obtained by
(i) preparing a mixture of tegaserod salt (prepared as above), diluent and lubricant,
(ii) sieving the mixture
(iii) adding the disintegrant, glidant, lubricant and optionally binder and blending the sieved mixture of step (ii) and
(iv) forming tablets by direct compression.

Part of the lubricant may be added in the mixture of step (i), the rest in the final mixture of step (iii) or the total amount of lubricant may be added in the final mixture of step (iii).

The resulting powder blends of step iii) are compressed on either a single punch press (Korsh EKO), 6 station-rotary press (Korsh PH106), 17 station-rotary press (Korsh PH 230) or 43 station-rotary press (Fette PT2090).

All components may be mixed together, sieved through and mixed again. Tablets are then formed by direct compression.

A 6 mg tablet is prepared using the direct compression method.

| Component | Quantitiy (125 mg tablet) % w/w |
|---|---|
| salt of 3-(5-methoxy-1H-indol-3-yl-methylene)-N-pentylcarbazimidamide | 6.65 |
| Lactose spray dried | 72.25 |
| hydroxy propylmethyl cellulose | 5 |
| Crospovidone | 10.00 |
| Aérosil | 0.10 |
| glyceryl behenate | 6.00 |

A preblend is formed by mixing the tegaserod salt, hydroxy propylmethyl cellulose, a part of glyceryl behenate and a part of lactose. This preblend is mixed with the remaining excipients except glyceryl behenate.This blend is lubricated with the remaining part of glyceryl behenate.
The final blend is compressed using a rotary press (Korsh PH 343 or Fette PT2090) equipped with 7 mm, round upper punches.

## Claims

1. A salt form of tegaserod, or a solvate thereof, prepared from an acid selected from: acetic acid; L-malic acid; naphtalene 1,5 disulfonic acid;
adipic acid phosphoric acid nicotinic acid L-ascorbic acid; succinic acid; oxalic acid; citric acid; orotic acid; aspartic acid; fumaric acid; thiocyanic acid; propionic acid; galactaric acid; salycilic acid; L-pyroglutamic acid; glutaric acid; benzenesulfonic acid; p-toluenesulfonic acid; glycolic acid; benzoic acid; camphor 10 sulfonic acid; glucuronic acid; (+) camphoric acid; formic acid; hippuric acid; ethansulfonic acid; 4-acetamidobenzoic; α-oxo-glutaric; gentisic acid; lactobionic acid; DL-lactic acid; malonic acid; sulfuric acid; squaric acid; deoxycholic acid; 1-hydroxy-naphtoic acid; phtalic acid; vanillic acid; methansulfonic acid; dichloroacetic acid; isobutyric acid; nitric acid; carbonic acid; and emboic acid.

2. A salt form of tegaserod prepared from an acid according to claim 1 which salt form is selected from: Tegaserod glycolate; Tegaserod D,L-lactate; Tegaserod L-lactate; Tegaserod acetate; Tegaserod citrate; Tegaserod sulfate; Tegaserod hydrogenmalate; Tegaserod malate; Tegaserod D-gluconate; Tegaserod glucuronate; Tegaserod salicylate; Tegaserod gentisate; Tegaserod hippurate; Tegaserod succinate; Tegaserod fumarate; Tegaserod adipate; Tegaserod thiocyanate; Tegaserod aspartate; Tegaserod malonate; Tegaserod galactarate; Tegaserod oxoglutarate; Tegaserod pidolate; Tegaserod glutarate; Tegaserod benzoate; Tegaserod oxalate; Tegaserod esylate; Tegaserod Mesylate; Tegaserod formate; Tegaserod quadrate; Tegaserod propionate; Tegaserod dichloroacetate; Tegaserod acetamidobenzoate; Tegaserod besylate; Tegaserod pyruvate; Tegaserod tosylate; Tegaserod isobutyrate; Tegaserod caprylate; Tegaserod napadisilate; Tegaserod xinafoate; Tegaserod pamoate; and Tegaserod nitrate.

3. A solvate of a salt form according to claim 1 or claim 2 which is selected from: Tegaserod hydrogencitrate monohydrate; Tegaserod sulfate monohydrate; Tegaserod D-gluconate hemihydrate; and Tegaserod galactarate monohydrate.

4. A pharmaceutical composition comprising a pharmaceutically acceptable carrier or diluent and a therapeutically effective amount of a salt according to any one of claims 1 to 3.

5. The use of a salt according to any of claims 1 to 3 for the manufacture of a medicament for the treatment of a disease in which 5HT₄ agonists or partial agonists are implicated.

6. The use of a salt according to any of claims 1 to 3 for the manufacture of a medicament for the treatment of irritable bowel syndrome, gastro-esophageal reflux disease, functional dyspepsia, chronic constipation or diarrhea.

7. A method of treating irritable bowel syndrome, gastro-esophageal reflux disease, functional dyspepsia, chronic constipation or diarrhea comprising administering to subject in need of such treatment a therapeutically effective amount of a salt according to any of claims 1 to 3.
